# EUROPEAN PATENT APPLICATION

(11) **EP 4 621 794 A1**
(43) Date of publication of application: **24.09.2025**
(21) Application number: 25164613.9
(22) Date of filing: 19.03.2025
(51) Int. Cl.: G16H 30/40, G16H 50/20, G16H 50/30

(54) **DATA PROCESSING APPARATUS, DATA PROCESSING METHOD, AND DATA PROCESSING PROGRAM**

(30) Priority: 22.03.2024 JP 2024046240
(71) Applicant: J. MORITA MANUFACTURING CORPORATION, Fushimi-ku, Kyoto-shi, Kyoto 6128533 (JP)
(72) Inventor: KAJI, Ryosuke, Kyoto-shi Kyoto 612-8533 (JP); WAKAZOME, Naonori, Kyoto-shi Kyoto 612-8533 (JP)
(74) Representative: Müller Hoffmann & Partner

(57) **Abstract**

A data processing apparatus includes: an input unit configured to acquire three-dimensional data including three-dimensional position information corresponding to each of a plurality of points representing a surface shape of intraoral objects including a tooth; a computing unit configured to perform a process for identifying each of a plurality of parts of the tooth, based on the three-dimensional data acquired by the input unit; and an output unit configured to output a result of the process performed by the computing unit.

## Description

### CROSS REFERENCE TO RELATED APPLICATIONS

This application is based on Japanese Patent Application No. 2024-046240 filed on March 22, 2024 with the Japan Patent Office.

### BACKGROUND

### Field

The present disclosure relates to a data processing apparatus, a data processing method, and a data processing program for processing three-dimensional data.

### Description of the Background Art

It is known that keeping teeth in a healthy condition is effective for extending the healthy life expectancy. In view of this, introduction of mandatory annual dental checkups for all the people, which is so-called universal dental checkup, is being considered. During a dental checkup, a practitioner such as dentist examines health conditions of teeth and gums of a patient, for example, and records the results of the examination in a medical chart. At this time, the practitioner is required to examine the health condition of a tooth while identifying the type of the tooth and the part of the tooth (surface part) and record, in the medical chart, the results of the examination for each type and each part of the tooth.

Regarding this, Japanese Patent Laying-Open No. 2020-96691 discloses a data processing apparatus that identifies the type of a tooth based on three-dimensional data including three-dimensional position information corresponding to each of a plurality of points constituting the tooth.

### SUMMARY

While a practitioner can identify the tooth type with high precision using the data processing apparatus disclosed in Japanese Patent Laying-Open No. 2020-96691, the practitioner is required to identify not only the type of the tooth to be examined but also the part of the tooth to be examined. Currently, however, the practitioner has to identify the part of the tooth by him or herself and, depending on the skills and abilities of the practitioner, there is a possibility that the dental checkup takes a long time and/or the tooth part where decay or the like has occurred is erroneously identified, resulting in a wrong record in the medical chart.

The present disclosure is given to solve such a problem, and an object of the present disclosure is to provide a technique that enables a tooth part to be identified easily with high precision.

In particular, a data processing apparatus configured to process three-dimensional data is provided. The data processing apparatus includes: an input unit configured to acquire the three-dimensional data including three-dimensional position information corresponding to each of a plurality of points representing a surface shape of intraoral objects including a tooth; a computing unit configured to perform a process for identifying each of a plurality of parts of the tooth, based on the three-dimensional data acquired by the input unit; and an output unit configured to output a result of the process performed by the computing unit.

Further, a data processing method of processing three-dimensional data by a computer is provided. The data processing method includes, as processing performed by the computer: acquiring the three-dimensional data including three-dimensional position information corresponding to each of a plurality of points representing a surface shape of intraoral objects including a tooth; performing a process for identifying each of a plurality of parts of the tooth, based on the three-dimensional data acquired in the acquiring; and outputting a result of the process performed in the performing.

Further, a data processing program for processing three-dimensional data by a computer is provided. The data processing program causes the computer to perform: acquiring the three-dimensional data including three-dimensional position information corresponding to each of a plurality of points representing a surface shape of intraoral objects including a tooth; performing a process for identifying each of a plurality of parts of the tooth, based on the three-dimensional data acquired in the acquiring; and outputting a result of the process performed in the performing.

The foregoing and other objects, features, aspects and advantages of the present disclosure will become more apparent from the following detailed description of the present disclosure when taken in conjunction with the accompanying drawings.

### BRIEF DESCRIPTION OF THE DRAWINGS

Fig. 1 is a diagram showing an application example of a data processing apparatus.
Fig. 2 is a block diagram showing a hardware configuration of the data processing apparatus.
Fig. 3 is a diagram for illustrating an example of parts of teeth.
Fig. 4 is a diagram for illustrating a modification of parts of each tooth that is a first molar, a second molar, a third molar, a first premolar, or a second premolar.
Fig. 5 is a diagram for illustrating a modification of parts of each tooth that is a canine, a lateral incisor, or a central incisor.
Fig. 6 is a block diagram showing a functional configuration of the data processing apparatus.
Fig. 7 is a diagram showing an example of the result of identification of parts of teeth acquired by the data processing apparatus.
Fig. 8 is a diagram showing an example of the result of identification of parts of teeth acquired by the data processing apparatus.
Fig. 9 is a diagram showing an example of the result of identification of parts of teeth acquired by the data processing apparatus.
Fig. 10 is a diagram showing an example of the result of identification of parts of teeth acquired by the data processing apparatus.
Fig. 11 is a diagram showing an example of data processing by the data processing apparatus using an estimation model.
Fig. 12 is a diagram showing an example of data processing by the data processing apparatus using an estimation model.
Fig. 13 is a diagram showing an example of data processing by the data processing apparatus using an estimation model.
Fig. 14 is a diagram showing a modification of a process of identifying parts of teeth by the data processing apparatus.
Fig. 15 is a diagram showing an example of the identification process by the data processing apparatus using a tooth surface extraction logic.
Fig. 16 is a diagram showing an example of the identification process by the data processing apparatus using a tooth surface extraction logic.
Fig. 17 is a diagram showing an example of the identification process by the data processing apparatus using a tooth surface extraction logic.
Fig. 18 is a diagram showing an example of the identification process by the data processing apparatus using a tooth surface extraction logic.
Fig. 19 is a diagram showing an example of the identification process by the data processing apparatus using a tooth surface extraction logic.
Fig. 20 is a diagram showing an example of the identification process by the data processing apparatus using a tooth surface extraction logic.
Fig. 21 is a diagram showing an example of the identification process by the data processing apparatus using a tooth surface extraction logic.
Fig. 22 is a diagram showing an example of the identification process by the data processing apparatus using a tooth surface extraction logic.
Fig. 23 is a diagram showing an example of the identification process by the data processing apparatus using a tooth surface extraction logic.
Fig. 24 is a diagram showing an example of the identification process by the data processing apparatus using a tooth surface extraction logic.
Fig. 25 is a diagram showing an example of the identification process by the data processing apparatus using a tooth surface extraction logic.
Fig. 26 is a diagram showing an example of the identification process by the data processing apparatus using a tooth surface extraction logic.
Fig. 27 is a diagram showing an example of the identification process by the data processing apparatus using a tooth surface extraction logic.
Fig. 28 is a diagram showing an example of the identification process by the data processing apparatus using a tooth surface extraction logic.
Fig. 29 is a flowchart defining data processing performed by the data processing apparatus.
Fig. 30 is a diagram showing an example electronic medical chart generated by the data processing apparatus.
Fig. 31 is a diagram showing an example electronic medical chart generated by the data processing apparatus.
Fig. 32 is a diagram showing an example electronic medical chart generated by the data processing apparatus.
Fig. 33 is a diagram showing an example electronic medical chart generated by the data processing apparatus.
Fig. 34 is a diagram showing an example electronic medical chart generated by the data processing apparatus.
Fig. 35 is a diagram showing an example electronic medical chart generated by the data processing apparatus.

### DESCRIPTION

The present disclosure is described in detail with reference to the drawings. In the drawings, the same or corresponding parts are denoted by the same reference characters, and a description thereof is not herein repeated.

### [Application Example]

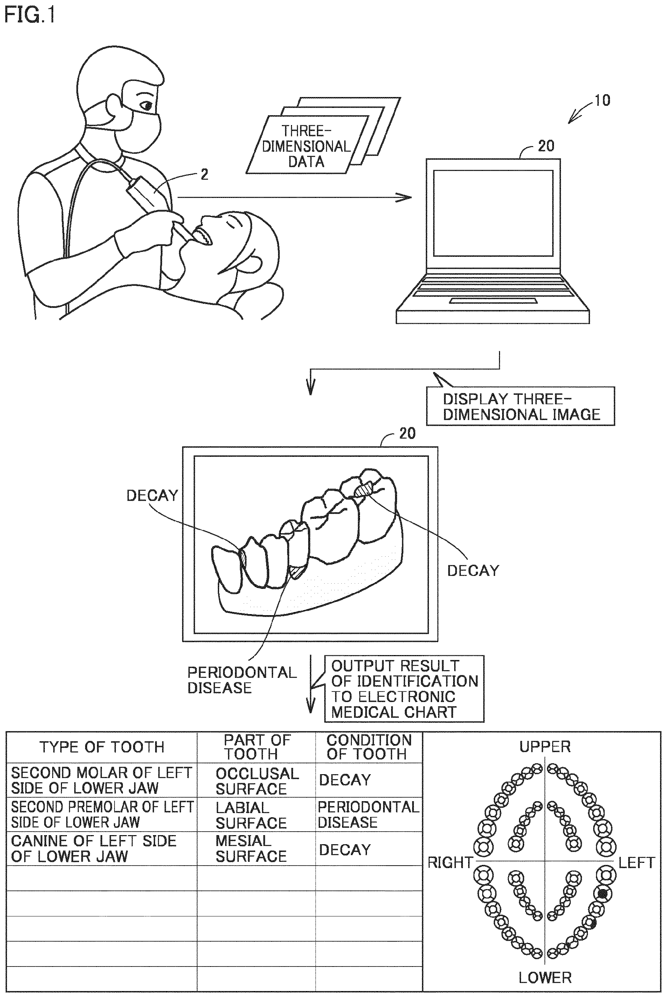

An application example of a data processing apparatus 10 is described with reference to Fig. 1. Fig. 1 is a diagram showing an application example of data processing apparatus 10.

A user can use a three-dimensional scanner 2 (for example, an optical scanner) to scan the inside of the oral cavity of a subject, and thereby acquire three-dimensional data on intraoral objects. "Intraoral objects" include biological tissues such as teeth and gums, and artificially-formed parts such as implant, cavity-prepared tooth, abutment tooth, and prosthesis. The three-dimensional data includes three-dimensional position information corresponding to each of a plurality of points (point cloud) representing a surface shape of intraoral objects. Specifically, the three-dimensional data includes coordinates (X, Y, Z) of each point in the point cloud representing a surface shape of intraoral objects, with respect to predetermined lateral direction (X-axis direction), longitudinal direction (Y-axis direction), and height direction (Z-axis direction). Further, the three-dimensional data includes color information indicating the actual color of a portion (a surface portion of an object) corresponding to each point of the point cloud representing a surface shape of intraoral objects.

The user may use a CT (Computed Tomography) imaging device (not shown) instead of three-dimensional scanner 2 to image the inside of the oral cavity of the subject. The CT imaging device includes a CBCT (Cone Beam Coherence Tomography) device for computed tomography of the subject's upper jaw and lower jaw by means of a cone beam (X-ray beam) in a conical shape. The user can use the CT imaging device to image the subject's upper jaw and lower jaw and thereby acquire three-dimensional volume (voxel) data on hard tissues (bones, teeth, and the like) other than soft tissues (skin, gums, and the like) around the subject's upper jaw and lower jaw. The user may also use an OCT (Optical Coherence Tomography) device for computed tomography of the subject's upper jaw and lower jaw. The user can perform tomographic processing on volume data on the imaged subject obtained by means of the CT imaging device or the OCT device, to generate a tomographic image or an appearance image of the subject. Further, the user can perform processing such as changing voxels into dots on the volume (voxel) data on a tooth acquired by means of the CT imaging device or the OCT device, to generate three-dimensional data including three-dimensional position information corresponding to each of a plurality of points (point cloud) representing the surface shape of the tooth.

As described above, the "three-dimensional data" includes at least one of three-dimensional scanner data (IOS (Intra Oral Scanner) data) acquired by scanning the tooth by means of three-dimensional scanner 2, CBCT data acquired by imaging the tooth by means of the cone beam CT, or OCT data acquired by imaging the tooth by means of the optical coherence tomography device.

The "user" includes practitioners (such as doctors) in various fields such as dentistry, oral surgery, orthopedics, plastic surgery, and cosmetic surgery, or assistants (such as dental assistant, dental hygienist, dental technician, nurse). Moreover, the "subject" includes patients of dentistry, oral surgery, orthopedics, plastic surgery, cosmetic surgery, and the like. Three-dimensional scanner 2 is a so-called intraoral scanner (IOS) capable of optically imaging the inside of the oral cavity of the patient by confocal method, triangulation method, or the like, and is capable of acquiring position information of each point of a point cloud representing the surface shape of an object to be scanned (for example, tooth and gum in the oral cavity) placed on a certain coordinate space.

Based on the three-dimensional data acquired by three-dimensional scanner 2, data processing apparatus 10 generates a rendered image (appearance image) indicating a surface shape of intraoral objects in three dimensions. The "rendered image" is an image generated by processing or editing certain data. For example, the user can perform processing or editing on the three-dimensional data on the object acquired by three-dimensional scanner 2, to generate a rendered image showing a two-dimensional object (a portion of the object that can be indicated by the IOS data) as seen from a predetermined viewpoint. Further, the user can change the predetermined viewpoint in multiple directions to generate a plurality of rendered images showing the two-dimensional object as seen in multiple directions.

Data processing apparatus 10 causes the rendered image generated based on the three-dimensional data acquired by three-dimensional scanner 2 to be presented on a display 20. The user can acquire the three-dimensional data on intraoral objects step by step, while viewing the rendered image presented on display 20.

During a dental checkup, the user such as a dentist or a dental hygienist examines health conditions of teeth and gums of a patient, i.e., a subject, and records the results of the examination in a medical chart. At this time, the user is required to examine the health condition of a tooth while identifying the type of the tooth and the part (surface part) of the tooth, and record the results of the examination in the medical chart for each tooth type and each tooth part. The user is therefore required to perform the dental checkup while identifying the type and the part of the tooth undergoing the examination. However, in the case where the user identifies the type and the part of the tooth by him or herself, there is a possibility that, depending on the skills and abilities of the user, the dental checkup takes a long time and/or or the tooth part where decay or the like has occurred is erroneously identified, resulting in a wrong record in the medical chart.

In view of this, data processing apparatus 10 uses AI (Artificial Intelligence) technology to identify the type and the part of the scanned tooth, based on the three-dimensional data indicating a surface shape of intraoral objects including the tooth.

The "type of the tooth" includes central incisor, lateral incisor, canine, first premolar, second premolar, first molar, second molar, and third molar of the right side of the upper jaw. The "type of the tooth" includes central incisor, lateral incisor, canine, first premolar, second premolar, first molar, second molar, and third molar of the left side of the upper jaw. The "type of the tooth" includes central incisor, lateral incisor, canine, first premolar, second premolar, first molar, second molar, and third molar of the right side of the lower jaw. The "type of the tooth" includes central incisor, lateral incisor, canine, first premolar, second premolar, first molar, second molar, and third molar of the left side of the lower jaw.

The "part of the tooth" includes respective parts into which the surface of each tooth as described above is divided. While details are described later herein, in the case of the second molar of the left side of the lower jaw, for example, the "part of the tooth" includes respective parts such as occlusal surface, distal surface, mesial surface, lingual surface, and buccal surface, generated by dividing the surface of the second molar of the left side of the lower jaw into a plurality of parts (occlusal surface, distal surface, mesial surface, lingual surface, and buccal surface).

Data processing apparatus 10 generates image data for displaying a diagram or a table indicating the results of the identification of the type of the tooth and the part of the tooth. The image data includes data for displaying the tooth in at least one of two dimensions and three dimensions, together with the results of the identification. Data processing apparatus 10 can output the image data to display 20 to thereby cause the diagram or the table indicating the results of the identification to be presented on display 20.

Further, data processing apparatus 10 may identify, based on the three-dimensional data, an artificially-formed part of a tooth, among intraoral objects having been scanned. The "artificially-formed part" includes implant, cavity-prepared tooth, abutment tooth, and prosthesis. The implant includes an implant fixture corresponding to the tooth root, a scan body, and an abutment corresponding to the abutment tooth portion. Further, to the implant fixture, the abutment, the abutment tooth, and the cavity-prepared tooth, inlay, onlay, crown, bridge, veneer, denture, and the like are applied.

Based on the three-dimensional data, data processing apparatus 10 may identify a lesion among intraoral objects (teeth and gums) having been scanned. The "lesion" is a part having a defect in the oral cavity, and includes, for example, a part where decay has occurred, a part where periodontal disease has occurred, a part where gingivitis has occurred, and a part where a tooth has been chipped. The "lesion" is not limited to a part that is actually diagnosed as a disease, but may include a part where a disease is likely to occur, such as a part where calculus accumulates or a part where plaque (dental plaque) adheres.

Data processing apparatus 10 may estimate the depth of a periodontal pocket in a tooth based on the three-dimensional data. Further, data processing apparatus 10 may estimate a premature contact position of teeth based on the three-dimensional data.

Various processes such as a process of identifying a type of a tooth, a process of identifying a part of a tooth, a process of identifying an artificially-formed part of a tooth, a process of identifying a lesion of a tooth, a process of estimating the depth of a periodontal pocket in a tooth, and a process of estimating a premature contact position of teeth, which are performed by data processing apparatus 10, are also collectively referred to as "data processing" hereinafter. The results obtained by the data processing (results of the identification and results of the estimation) are also collectively referred to as "processing results" hereinafter.

Data processing apparatus 10 may further add, to the diagram or the table indicating the results of identification of the type of the tooth and the part of the tooth, at least one piece of information among artificially-formed part, lesion, the depth of a periodontal pocket, and premature contact position. Data processing apparatus 10 can output image data indicating these processing results to display 20, to thereby present, on display 20, an image indicating the processing results such as a lesion, an artificially-formed part, the depth of a periodontal pocket, or a premature contact position obtained for each tooth type and each tooth part.

Data processing apparatus 10 can also record processing results such as a lesion, an artificially-formed part, the depth of a periodontal pocket, or a premature contact position, obtained for each tooth type and each tooth part, in the form of an electronic medical chart in a storage device 13 described later herein, or can transmit the processing results to an external server device via a communication device 18. Data processing apparatus 10 may also record, in storage device 13, processing results such as a lesion, an artificially-formed part, the depth of a periodontal pocket, or a premature contact position, obtained for each tooth type and each tooth part, not only in the form of an electronic medical chart, but also in the form of a hygienist work record for recording a work of a dental hygienist, or a document relating to a dental hygienist guidance for recording details of guidance for dental hygienists, or can transmit the processing results to an external server device via communication device 18.

For example, as shown in Fig. 1, data processing apparatus 10 presents, on display 20, an image indicating the surface shape of a part of the dentition, and indicates, on the image presented on display 20, a colored part where decay identified by the data processing occurs, or a colored part of gums where periodontal disease identified by the data processing occurs. Data processing apparatus 10 also records, in the form of an electronic medical chart, the type of the tooth and the part of the tooth where a lesion such as decay or periodontal disease identified by the data processing occurs, and presents the processing results on display 20 using a diagram or a table.

Thus, data processing apparatus 10 can automatically make diagnose and/or suggestion about various examinations for the type of the tooth, the part of the tooth, an artificially-formed part, a lesion, the depth of a periodontal pocket, and a premature contact position, for example, based on the three-dimensional data. Accordingly, the user is not required to identify, by him or herself, the type of the tooth or the part of the tooth by viewing the image indicating the processing results presented on display 20 or by viewing, through display 20, an electronic medical chart in which the processing results are recorded, or required to perform, by him or herself, various kinds of examinations for an artificially-formed part, a lesion, the depth of a periodontal pocket, or a premature contact position. Therefore, it is possible for the user to avoid taking time for a dental checkup or avoid erroneously identifying the tooth part where decay for example has occurred and thereby avoid recording wrong information in a medical chart.

### [Hardware Configuration of Data Processing Apparatus]

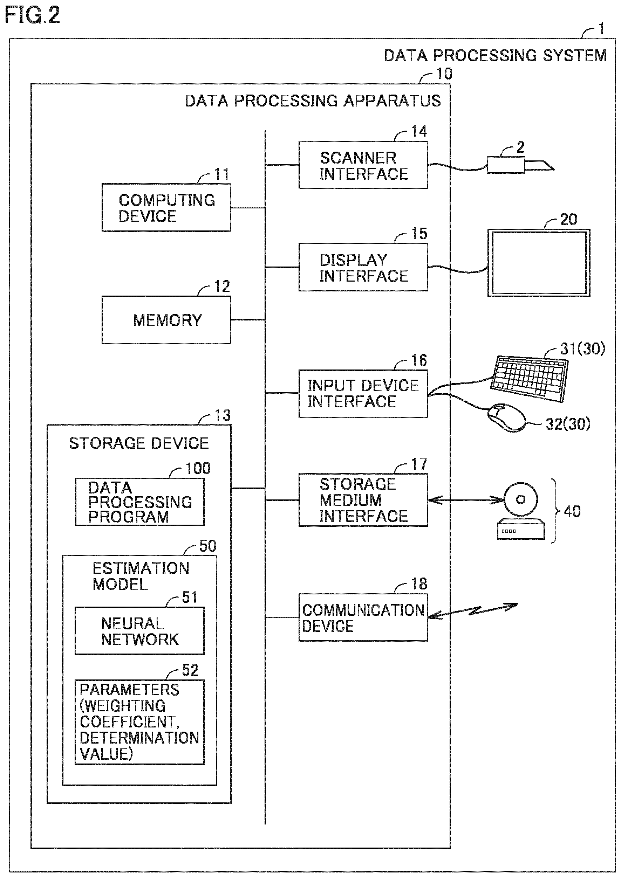

A hardware configuration of data processing apparatus 10 is described with reference to Fig. 2. Fig. 2 is a block diagram showing the hardware configuration of data processing apparatus 10. Data processing apparatus 10 may for example be implemented by a general-purpose computer or may be implemented by a computer dedicated to three-dimensional scanner 2.

As shown in Fig. 2, data processing system 1 includes data processing apparatus 10, three-dimensional scanner 2, display 20, and an input device 30 such as a keyboard 31 and a mouse 32. Data processing apparatus 10 includes, as main hardware elements, a computing device 11, a memory 12, a storage device 13, a scanner interface 14, a display interface 15, an input device interface 16, a storage medium interface 17, and a communication device 18.

Computing device 11 is a computing entity (computer) that executes various programs to execute various processes, and is an example of the "computing unit." Computing device 11 is configured as a processor such as CPU (Central Processing Unit), MPU (Micro Processing Unit), TPU (Tensor Processing Unit), or GPU (Graphics Processing Unit). While the processor which is an example of computing device 11 has functions of executing predetermined processes by executing predetermined programs, some or all of these functions may be implemented with a dedicated hardware circuit such as ASIC (Application Specific Integrated Circuit) or FPGA (Field Programmable Gate Array). The "processor" is not limited to a processor in a narrow sense such as CPU, MPU, TPU, or GPU that performs processes by the stored program method, and may include a hardwired circuit such as ASIC or FPGA. Moreover, computing device 11 is not limited to the von Neumann computer such as CPU or GPU, but may be configured as a non-von-Neumann computer such as quantum computer or optical computer. Computing device 11 as described above can also be read as processing circuitry that executes a predetermined process. Computing device 11 may be configured with one chip or may be configured with a plurality of chips. Further, the processor and associated processing circuitry may be constituted of a plurality of computers interconnected by wired or wireless connection via a local area network or wireless network, for example. The processor and associated processing circuitry may also be configured as cloud computers that remotely perform computation based on input data and output the results of the computation to other remote devices.

Memory 12 includes a volatile storage area (for example, a working area) that temporarily stores program codes and/or work memory, for example, when computing device 11 executes various programs. Examples of the storage include volatile memories such as DRAM (dynamic random access memory) and SRAM (static random access memory), and nonvolatile memories such as ROM (Read Only Memory) and flash memory.

Storage device 13 stores various programs executed by computing device 11, various data, and the like. Storage device 13 may be one or a plurality of non-transitory computer-readable media or may be one or a plurality of computer-readable storage media. Examples of storage device 13 include hard disk drive (HDD) and solid state drive (SSD), for example.

Storage device 13 stores a data processing program 100 and an estimation model 50. In data processing program 100, details of data processing for computing device 11 to identify a type of a tooth or identify a part of a tooth using estimation model 50 based on three-dimensional data indicating a surface shape of intraoral objects are written.

Estimation model 50 includes a neural network 51 and parameters 52 used by neural network 51.

Neural network 51 is trained by machine learning to execute various types of data processing based on three-dimensional data on intraoral objects. Specifically, neural network 51 is trained by machine learning (for example, supervised learning) to identify a type of a tooth based on the three-dimensional data that is directly input to the neural network. Neural network 51 is trained by machine learning (for example, supervised learning) to identify each of a plurality of parts of a tooth based on the three-dimensional data that is directly input to the neural network. "The three-dimensional data is directly input" is herein not limited to that the three-dimensional data acquired by three-dimensional scanner 2 is input as it is to neural network 51 without being modified, but also includes that the three-dimensional data acquired by three-dimensional scanner 2 that may be modified slightly by preprocessing, which is substantially the same as the exact three-dimensional data acquired by three-dimensional scanner 2, is input to neural network 51. Neural network 51 is trained by machine learning (for example, supervised learning) to identify an artificially-formed part of a tooth, based on the three-dimensional data that is directly input. Neural network 51 is trained by machine learning (for example, supervised learning) to identify a lesion of a tooth based on the three-dimensional data that is directly input. Neural network 51 is trained by machine learning (for example, supervised learning) to estimate the depth of a periodontal pocket in a tooth based on the three-dimensional data that is directly input.

Any algorithm may be applied to neural network 51 as long as the algorithm is applicable to neural network 51 of the embodiments, such as auto encoder, convolutional neural network (CNN), recurrent neural network (RNN), transformer, or generative adversarial network (GAN). Estimation model 50 may include, besides neural network 51, any of other known algorithms such as Bayesian estimation or support vector machine (SVM).

Parameters 52 include a weighting coefficient used for computation by neural network 51, and a determination value used for determination during the computation.

Scanner interface 14 is an example of the "input unit." Scanner interface 14 acquires three-dimensional data indicating a surface shape of intraoral objects. For example, scanner interface 14 is communicably connected to three-dimensional scanner 2 and acquires three-dimensional data from three-dimensional scanner 2. Scanner interface 14 may be communicably connected to a CT imaging apparatus (not shown) to acquire three-dimensional data indicating the surface shape of a tooth generated based on volume (voxel) data on the tooth acquired by the CT imaging apparatus. The three-dimensional data input from scanner interface 14 is stored in memory 12 or storage device 13, and is used by computing device 11 for performing data processing.

Display interface 15 is an interface for connecting display 20, and is an example of the "output unit." Display interface 15 allows data to be input and output between data processing apparatus 10 and display 20. For example, data processing apparatus 10 outputs, to display 20 via display interface 15, image data for presenting a diagram or a table showing the results of identification of a type of a tooth and a part of the tooth. Based on the image data received via display interface 15, display 20 presents the diagram or the table showing the results of identification of the type of the tooth and the part of the tooth.

Input device interface 16 is an interface for connecting input device 30 such as keyboard 31 and mouse 32. Input device interface 16 allows data to be input and output between data processing apparatus 10 and input device 30. For example, the user can use input device 30 to input a command signal for moving a cursor on the diagram or the table presented on display 20 or for processing the diagram or the table. Data processing apparatus 10 performs a process based on the command signal received via input device interface 16.

Storage medium interface 17 reads various kinds of data stored in a removable disk 40, which is a storage medium, and writes various kinds of data in removable disk 40. For example, storage medium interface 17 may acquire data processing program 100 from removable disk 40, or may write, in removable disk 40, data on an electronic medical chart generated by computing device 11. Removable disk 40 may be one or a plurality of non-transitory computer-readable media or may be one or a plurality of computer-readable storage media. In the case where computing device 11 acquires, from removable disk 40 via storage medium interface 17, three-dimensional data indicating a surface shape of intraoral objects, storage medium interface 17 may be an example of the "input unit." In the case where computing device 11 outputs data on an electronic medical chart to removable disk 40 via storage medium interface 17, storage medium interface 17 may be an example of the "output unit."

Communication device 18 transmits and receives data to and from an external device via wired communication or wireless communication. For example, communication device 18 acquires, from an external device, three-dimensional data indicating a surface shape of intraoral objects. The three-dimensional data acquired by communication device 18 is stored in memory 12 or storage device 13, and is used by computing device 11 for performing data processing. Communication device 18 may output data on an electronic medical chart generated by computing device 11 to an external device. In the case where computing device 11 acquires three-dimensional data indicating a surface shape of intraoral objects from an external device via communication device 18, communication device 18 may be an example of the "input unit." In the case where computing device 11 outputs data on an electronic medical chart to an external device via communication device 18, communication device 18 may be an example of the "output unit."

### [Parts of Tooth]

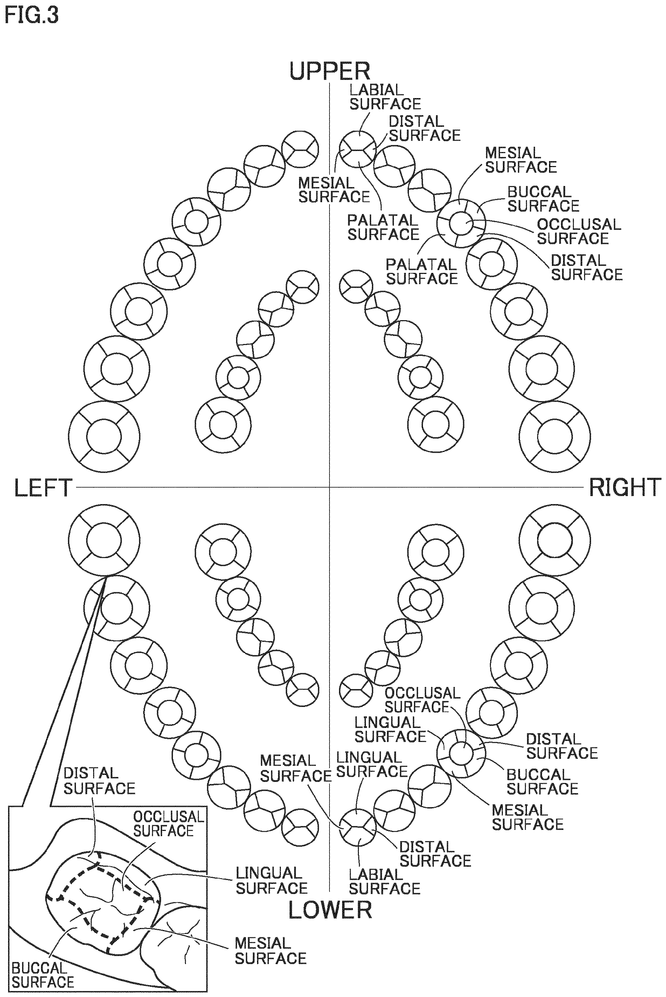

With reference to Figs. 3 to 5, each of a plurality of parts forming the surface of a tooth that are identified by data processing apparatus 10 is described. Fig. 3 is a diagram for illustrating an example for parts of teeth.

It is assumed that the surface of each tooth in the oral cavity is divided into a plurality of parts appropriately for each tooth type, as shown in Fig. 3. Regarding the first molars, the second molars, the third molars, the first premolars, or the second premolars of the upper jaw, the parts of each tooth include at least one of an occlusal surface, a distal surface, a mesial surface, a palatal surface, and a buccal surface. Regarding the canines, the lateral incisors, or the central incisors of the upper jaw, the parts of each tooth include at least one of a distal surface, a mesial surface, a palatal surface, and a labial surface. Regarding the first molars, the second molars, the third molars, the first premolars, or the second premolars of the lower jaw, the parts of each tooth include at least one of an occlusal surface, a distal surface, a mesial surface, a lingual surface, and a buccal surface. Regarding the canines, the lateral incisors, or the central incisors of the lower jaw, the parts of each tooth include at least one of a distal surface, a mesial surface, a lingual surface, and a labial surface.

Specifically, in the example shown in Fig. 3, regarding the first molar, the second molar, the third molar, the first premolar, and the second premolar of the right side or the left side of the upper jaw, the surface of each tooth is divided into five surfaces that are an occlusal surface, a distal surface, a mesial surface, a palatal surface, and a buccal surface. Regarding the canine, the lateral incisor, and the central incisor of the right side or the left side of the upper jaw, the surface of each tooth is divided into four surfaces that are a distal surface, a mesial surface, a palatal surface, and a labial surface. Regarding the first molar, the second molar, the third molar, the first premolar, and the second premolar of the right side or the left side of the lower jaw, the surface of each tooth is divided into five surfaces that are an occlusal surface, a distal surface, a mesial surface, a lingual surface, and a buccal surface. Regarding the canine, the lateral incisor, and the central incisor of the right side or the left side of the lower jaw, the surface of each tooth is divided into four surfaces that are a distal surface, a mesial surface, a lingual surface, and a labial surface.

Here, respective surfaces of the first premolar, the second premolar, the first molar, the second molar, and the third molar of the upper jaw and respective surfaces of the first premolar, the second premolar, the first molar, the second molar, and the third molar of the lower jaw that are to touch each other respectively are referred to as occlusal surface. The direction away from the front of the dental arch (away from the location between the left central incisor and the right central incisor) is referred to as distal, and a surface in the distal direction is referred to as distal surface. The direction toward the front of the dental arch is referred to as mesial, and a surface in the mesial direction is referred to as mesial surface. In the dental arch of the upper jaw, a side closer to the palate is referred to as palatal side, and a surface on the palatal side is referred to as palatal surface. In the dental arch of the lower jaw, a side closer to the tongue is referred to as lingual side, and a surface on the lingual side is referred to as lingual surface. In the dental arch of the upper jaw or the lower jaw, the side closer to the lips is referred to as labial side, and a surface on the labial side is referred to as labial surface. In the dental arch of the upper jaw or the lower jaw, the sides closer to the cheeks are referred to as buccal side, and a surface on the buccal side is referred to as buccal surface.

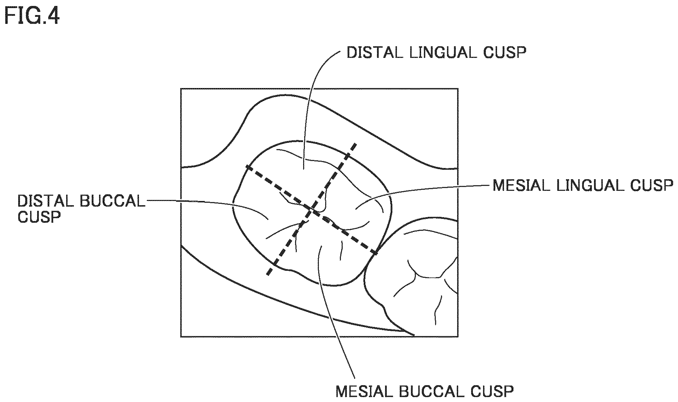

Regarding the first molar, the second molar, the third molar, the first premolar, or the second premolar, the definition of the parts is not limited to the example shown in Fig. 3, and parts of each tooth may also be defined as shown in Fig. 4. Fig. 4 is a diagram for illustrating a modification of parts of each tooth that is a first molar, a second molar, a third molar, a first premolar, or a second premolar.

Regarding the first molar, the second molar, the third molar, the first premolar, or the second premolar of the upper jaw, the parts of each tooth include a plurality of cusps forming the tooth. Similarly, regarding the first molar, the second molar, the third molar, the first premolar, or the second premolar of the lower jaw, the parts of each tooth include a plurality of cusps forming the tooth.

For example, as shown in Fig. 4, the surface of the second molar of the left side of the lower jaw is divided into four cusps that are a distal buccal cusp, a distal lingual cusp, a mesial buccal cusp, and a mesial lingual cusp. Similarly, the surface of the second molar of the right side of the lower jaw is divided into four cusps that are a distal buccal cusp, a distal lingual cusp, a mesial buccal cusp, and a mesial lingual cusp. Although not shown, the surface of the first molar of the left side of the upper jaw is divided into four cusps that are a distal buccal cusp, a distal palatal cusp, a mesial buccal cusp, and a mesial palatal cusp. Similarly, the surface of the first molar of the right side of the upper jaw is divided into four cusps that are a distal buccal cusp, a distal palatal cusp, a mesial buccal cusp, and a mesial palatal cusp.

Here, a cusp on the distal side and closer to the cheek is referred to as distal buccal cusp. A cusp on the distal side and closer to the tongue is referred to as distal lingual cusp. A cusp on the mesial side and closer to the cheek is referred to as mesial buccal cusp. A cusp on the mesial side and closer to the tongue is referred to as mesial lingual cusp. A cusp on the distal side and closer to the palate is referred to as distal palatal cusp. A cusp on the mesial side and closer to the palate is referred to as mesial palatal cusp.

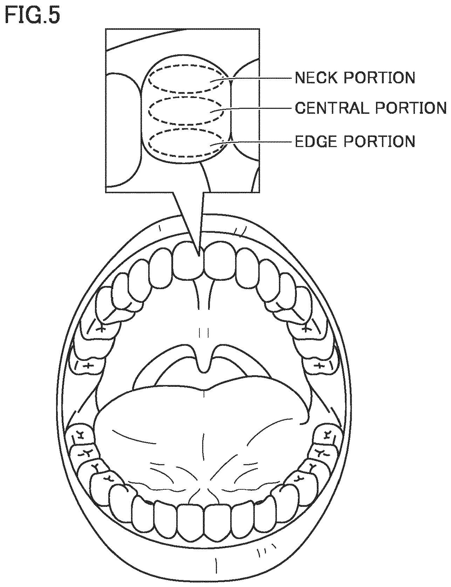

Not only the first molars and the second molars, but also other teeth such as the third molars, the first premolars, or the second premolars may include cusps. For example, the tip of the canine may be referred to as cusp. The number of cusps of the molar is not limited to four, but may be two to five, for example. The canine, the lateral incisor, or the central incisor is not limited to the example shown in Fig. 3, but parts of each tooth may be defined as shown in Fig. 5. Fig. 5 is a diagram for illustrating a modification of parts of each tooth that is a canine, a lateral incisor, or a central incisor.

As shown in Fig. 5, regarding the canines, the lateral incisors, or the central incisors of the upper jaw, the parts of each tooth include a plurality of surfaces forming the labial surface. Similarly, regarding the canines, the lateral incisors, or the central incisors of the lower jaw, the parts of each tooth include a plurality of surfaces forming the labial surface.

In the example shown in Fig. 5, regarding the canines, the lateral incisors, or the central incisors of the upper jaw, the labial surface of each tooth is divided into three surfaces that are a neck portion, a central portion, and an edge portion. Similarly, regarding the canines, the lateral incisors, or the central incisors of the lower jaw, the labial surface of each tooth is divided into three surfaces that are a neck portion, a central portion, and an edge portion.

Here, a portion of the labial surface that is closer to the gum is referred to as neck portion. A portion of the labial surface that is closer to the tip of the tooth is referred to as edge portion. A portion of the labial surface located between the neck portion and the edge portion is referred to as central portion.

As shown in Figs. 3 to 5, the user can use input device 30 or the like to define parts of a tooth. The way to define parts of a tooth shown in each of Figs. 3 to 5 is merely an example, and the user may define a plurality of parts of a tooth in another way, or may combine some parts of the teeth shown in Figs. 3 to 5.

### [Functional Configuration of Data Processing Apparatus]

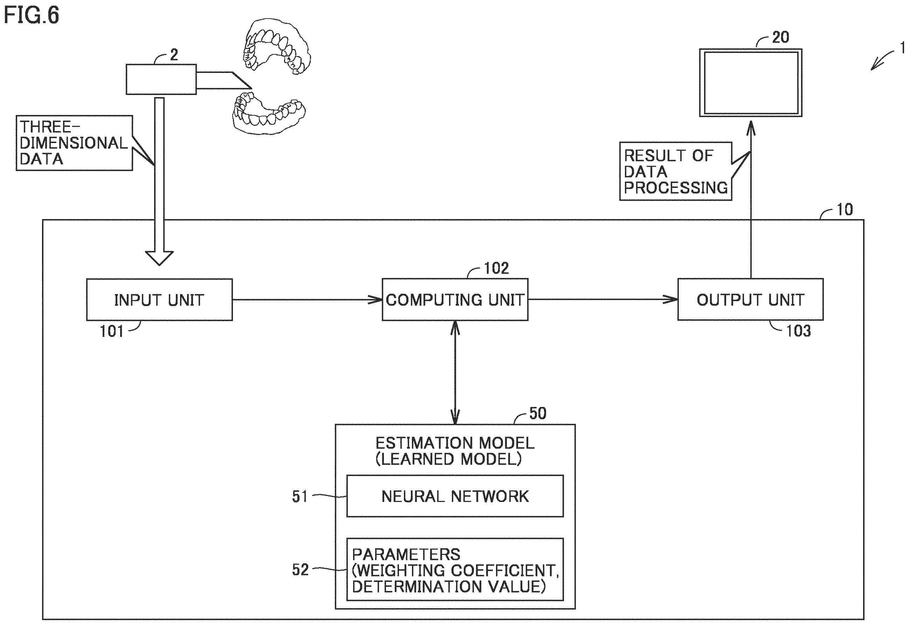

With reference to Fig. 6, a functional configuration of data processing apparatus 10 is described. Fig. 6 is a block diagram showing the functional configuration of data processing apparatus 10.

As shown in Fig. 6, data processing apparatus 10 includes an input unit 101, a computing unit 102, and an output unit 103, as functional units involved in data processing. Each of these functions is implemented through execution of data processing program 100 by computing device 11 of data processing apparatus 10.

Input unit 101 is a functional unit implemented by at least one of scanner interface 14, storage medium interface 17, and communication device 18. Three-dimensional data indicating a surface shape of intraoral objects is input to input unit 101.

Computing unit 102 is a functional unit implemented by computing device 11. Computing unit 102 performs predetermined data processing on the three-dimensional data input from input unit 101.

For example, based on the three-dimensional data input from input unit 101, and based on estimation model 50 for which machine learning has been performed to identify, based on the three-dimensional data, each of a plurality of parts of a tooth on which the three-dimensional data has been acquired, computing unit 102 identifies each of a plurality of parts of the tooth. Specifically, computing unit 102 identifies which one of the teeth of the upper jaw and the lower jaw has each of a plurality of parts of the tooth on which the three-dimensional data has been acquired.

More specifically, in the case where a tooth to be identified is the first molar, the second molar, the third molar, the first premolar, or the second premolar of the upper jaw, computing unit 102 uses estimation model 50 to estimate which of the occlusal surface, the distal surface, the mesial surface, the palatal surface, and the buccal surface corresponds to each of a plurality of parts forming the surface of the tooth to be identified. In the case where a tooth to be identified is the canine, the lateral incisor, or the central incisor of the upper jaw, computing unit 102 uses estimation model 50 to estimate which of the distal surface, the mesial surface, the palatal surface, and the labial surface corresponds to each of a plurality of parts forming the surface of the tooth to be identified. In the case where a tooth to be identified is the first molar, the second molar, the third molar, the first premolar, or the second premolar of the lower jaw, computing unit 102 uses estimation model 50 to estimate which of the occlusal surface, the distal surface, the mesial surface, the lingual surface, and the buccal surface corresponds to each of a plurality of parts forming the surface of the tooth to be identified. In the case where a tooth to be identified is the canine, the lateral incisor, or the central incisor of the lower jaw, computing unit 102 uses estimation model 50 to estimate which of the distal surface, the mesial surface, the lingual surface, and the labial surface corresponds to each of a plurality of parts forming the surface of the tooth to be identified.

Moreover, based on the three-dimensional data input from input unit 101 and based on estimation model 50 for which machine learning has been performed to identify the type of a tooth on which the three-dimensional data has been acquired, computing unit 102 identifies the type of the tooth.

Based on the three-dimensional data input from input unit 101 and based on estimation model 50 for which machine learning has been performed to identify an artificially-formed part of a tooth on which the three-dimensional data has been acquired, computing unit 102 identifies an artificially-formed part of the tooth.

Based on the three-dimensional data input from input unit 101 and based on estimation model 50 for which machine learning has been performed to identify a lesion of a tooth or gum on which the three-dimensional data has been acquired, computing unit 102 identifies a lesion of the tooth or gum.

Based on the three-dimensional data input from input unit 101 and based on estimation model 50 for which machine learning has been performed to estimate the depth of a periodontal pocket in a tooth on which the three-dimensional data has been acquired, computing unit 102 estimates the depth of a periodontal pocket in the tooth.

Based on the three-dimensional data input from input unit 101, computing unit 102 estimates a premature contact position where the upper and lower dental arches make premature contact when they are occluded.

Output unit 103 is a functional unit implemented by at least one of display interface 15, storage medium interface 17, and communication device 18. Output unit 103 outputs, to display 20, image data for presenting a diagram or a table showing the results of respective data processing processes, such as the result of identification of the tooth part, the result of identification of the tooth type, the result of identification of an artificially-formed part, the result of identification of a lesion, the result of estimation of the depth of a periodontal pocket, and the result of estimation of a premature contact position that are acquired by computing unit 102, records the results of respective data processing processes in the form of an electronic medical chart, and/or output the recorded electronic medical chart to removable disk 40 or an external device.

Data processing apparatus 10 having the above-described various functional units can identify a part of a tooth by means of computing unit 102 based on the three-dimensional data input to input unit 101, and output the results of identification by means of output unit 103. Accordingly, the user can easily identify a part of a tooth with high precision, using data processing apparatus 10.

Further, based on the three-dimensional data input to input unit 101, data processing apparatus 10 can also calculate, by means of computing unit 102, the result of identification of the tooth type, the result of identification of an artificially-formed part, the result of identification of a lesion, the result of estimation of the depth of a periodontal pocket, or the result of estimation of a premature contact position, and can output the results of the data processing, by means of output unit 103. Thus, the user can shorten the time for a dental checkup and improve the accuracy of the dental checkup, without being required to specify, by him or herself, not only the tooth part but also the tooth type, an artificially-formed part, a lesion, the depth of a periodontal pocket, a premature contact position, or the like.

Moreover, each time a tooth is scanned with three-dimensional scanner 2, the three-dimensional data may be input to input unit 101 and, each time the three-dimensional data is input to input unit 101, computing unit 102 may use the three-dimensional data to calculate the result of identification of the tooth part, the result of identification of the tooth type, the result of identification of an artificially-formed part, the result of identification of a lesion, the result of estimation of the depth of a periodontal pocket, or the result of estimation of a premature contact position, and output unit 103 may output in real time the results of these data processing processes acquired during scanning by three-dimensional scanner 2. Thus, each time a tooth is scanned with three-dimensional scanner 2 during a dental checkup, the user can acquire the result of data processing in real time.

### [Example of Result of Identification of Tooth Part]

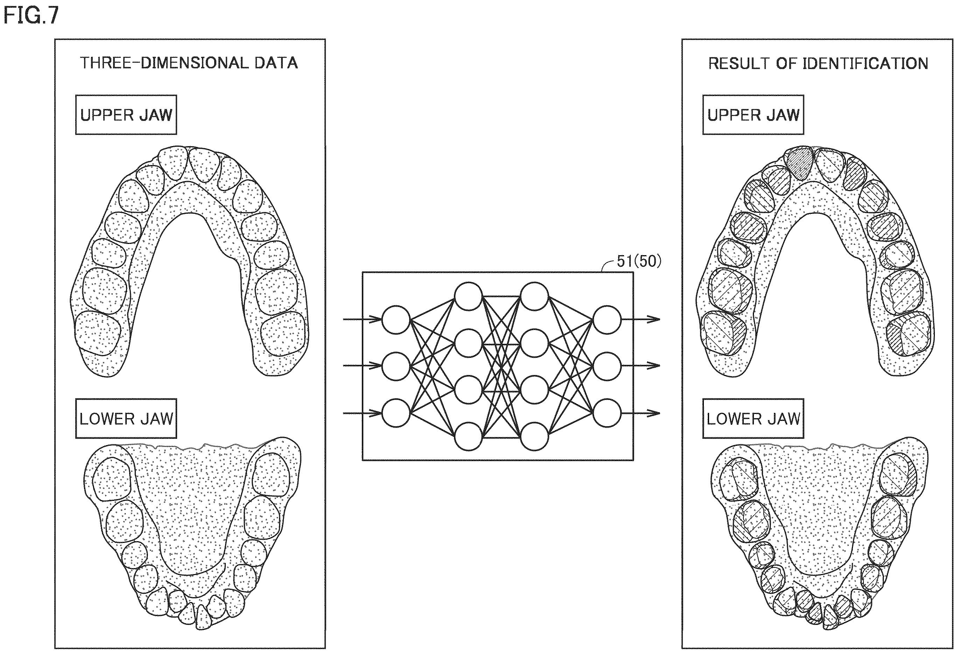

With reference to Figs. 7 to 10, the result of identification of parts of teeth acquired by data processing apparatus 10 is described. Figs. 7 to 10 are each a diagram showing an example of the result of identification of parts of teeth acquired by data processing apparatus 10.

Data processing apparatus 10 directly inputs, to neural network 51 of estimation model 50, three-dimensional data on each of teeth included in the dental arch of the upper jaw and the dental arch of the lower jaw, as shown in Figs. 7 to 10. Neural network 51 has undergone machine learning to identify each of the teeth based on the input three-dimensional data. For training of estimation model 50 by supervised learning, learning data is used that includes three-dimensional data indicating the surface shape of each tooth and colors associated in advance with a plurality of parts forming the surface of the tooth indicated by the three-dimensional data. Such coloring is not necessarily required, and any identification label may be used as long as it is a label that enables identification of a plurality of parts forming the surface of the tooth indicated by the three-dimensional data and, for example, numerical values that enable identification of a plurality of parts forming the surface of the tooth indicated by the three-dimensional data may be associated with these parts.

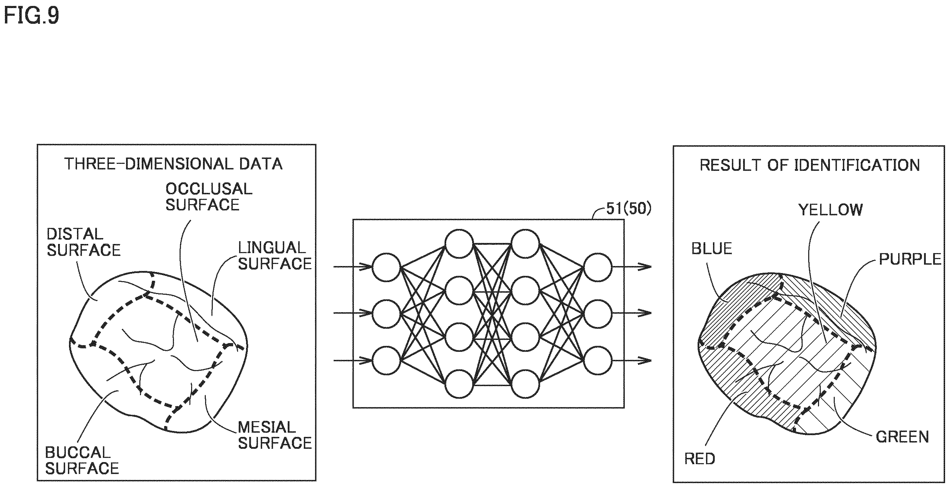

For example, as shown in Fig. 9, in the case of the second molar of the left side of the lower jaw, data indicating yellow is associated with the three-dimensional data corresponding to the occlusal surface, data indicating blue is associated with the three-dimensional data corresponding to the distal surface, data indicating red is associated with the three-dimensional data corresponding to the buccal surface, and data indicating green is associated with the three-dimensional data corresponding to the mesial surface. Although not shown, in the case of the second molar of the right side of the lower jaw, data indicating orange is associated with the three-dimensional data corresponding to the occlusal surface, data indicating navy blue is associated with the three-dimensional data corresponding to the distal surface, data indicating pink is associated with the three-dimensional data corresponding to the buccal surface, and data indicating yellow-green is associated with the three-dimensional data corresponding to the mesial surface. That is, in the learning data, the three-dimensional data corresponding to parts of each tooth that is input data are associated with respective different colors as correct answer data.

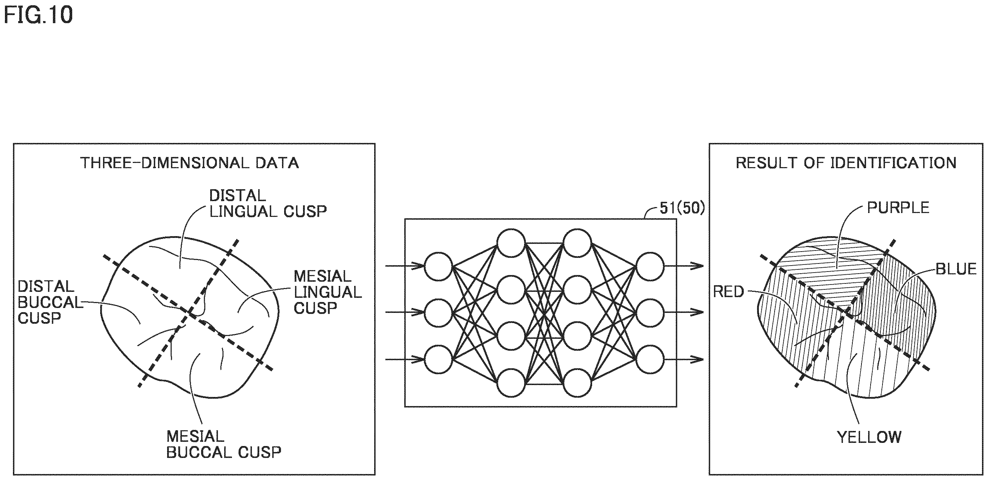

Regarding the first molar, the second molar, the third molar, the first premolar, and the second premolar, a predetermined color may be associated with the three-dimensional data corresponding to each of a plurality of cusps forming each tooth. For example, as shown in Fig. 10, in the case of the second molar of the left side of the lower jaw, data indicating red may be associated with the three-dimensional data corresponding to the distal buccal cusp, data indicating purple may be associated with the three-dimensional data corresponding to the distal lingual cusp, data indicating yellow may be associated with the three-dimensional data corresponding to the mesial buccal cusp, and data indicating blue may be associated with the three-dimensional data corresponding to the mesial lingual cusp. Although not shown, in the case of the second molar of the right side of the lower jaw, data indicating orange may be associated with the three-dimensional data corresponding to the distal buccal cusp, data indicating pink may be associated with the three-dimensional data corresponding to the distal lingual cusp, data indicating yellow-green may be associated with the three-dimensional data corresponding to the mesial buccal cusp, and data indicating navy blue may be associated with the three-dimensional data corresponding to the mesial lingual cusp. That is, in the learning data, the three-dimensional data corresponding to cusps of each tooth that is input data are associated with respective different colors as correct answer data.

In a training phase, estimation model 50 finds features of the surface shape of an object indicated by three-dimensional data input to neural network 51, estimates which part of which type of tooth to which the three-dimensional data corresponds, and outputs the color associated with the estimated part as the result of identification. Estimation model 50 determines whether or not the color of the output result of identification matches the color of the correct answer data and, if they do not match, optimizes parameters 52 in such a manner that makes these colors match each other. Accordingly, in an operation phase, estimation model 50 can estimate with high precision, based on the three-dimensional data input to neural network 51, the color of a part corresponding to the three-dimensional data, of a tooth of the type corresponding to the three-dimensional data, and output the estimated color as the result of identification.

For example, as shown in Fig. 7, in the case where the surface shape of the upper and lower dental arches on which the three-dimensional data has been acquired is presented on display 20 by a plurality of points (point cloud) having the three-dimensional data, data processing apparatus 10 uses estimation model 50 to estimate the color of each part based on the three-dimensional data, adds the estimated color to the points corresponding to each part, and further presents, on display 20, the surface shape of the upper and lower dental arches indicated by the colored points.

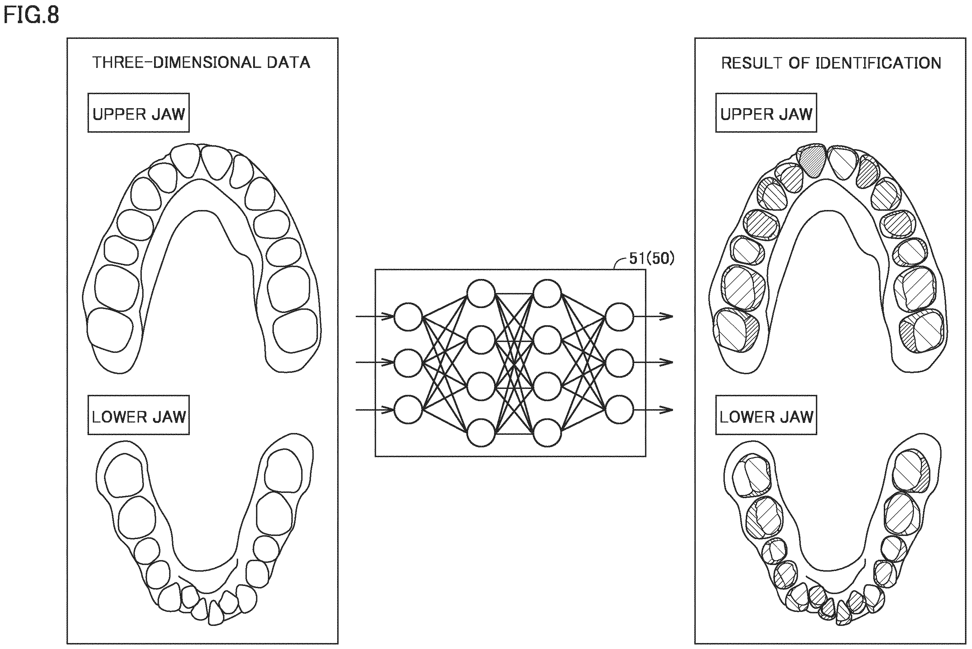

For example, as shown in Fig. 8, in the case where the surface shape of the upper and lower dental arches on which the three-dimensional data has been acquired is presented on display 20 by polygon meshes having the three-dimensional data, data processing apparatus 10 uses estimation model 50 to estimate the color of each part based on the three-dimensional data, adds the estimated color to the polygon mesh corresponding to each part, and further presents, on display 20, the surface shape of the upper and lower dental arches indicated by the colored polygon meshes.

Data processing apparatus 10 is not limited to identifying a part of each tooth based on the three-dimensional data on teeth such as dentition, but may identify a part of one tooth based on the three-dimensional data on the one tooth.

For example, as shown in Figs. 9 and 10, in the case where the surface shape of one tooth on which the three-dimensional data has been acquired is presented on display 20, data processing apparatus 10 uses estimation model 50 to estimate respective colors of the parts forming the surface of the one tooth, based on the three-dimensional data, and presents, on display 20, an image of the one tooth where the estimated colors are added to respective parts.

Thus, data processing apparatus 10 can identify the parts of a tooth based on the three-dimensional data using estimation model 50, and present the result of identification using colors. Accordingly, the user can easily identify the parts of the tooth with high precision, using data processing apparatus 10.

### [Example of Data Processing]

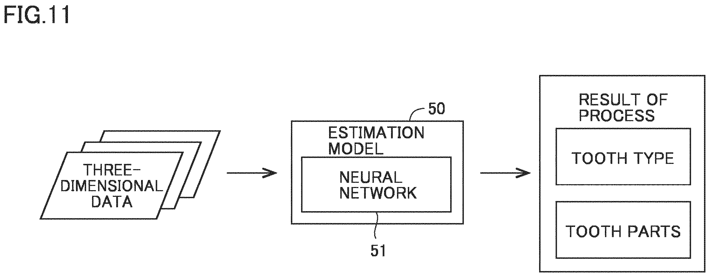

With reference to Figs. 11 to 13, an example of data processing performed by data processing apparatus 10 is described. Figs. 11 to 13 are each a diagram showing an example of data processing by data processing apparatus 10 using estimation model 50.

As shown in Fig. 11, data processing apparatus 10 may use one estimation model 50 to estimate the type and the part of a tooth based on the three-dimensional data. In this case, neural network 51 of estimation model 50 has been trained by machine learning (for example, supervised learning) to identify both the type and the part of a tooth based on the directly input three-dimensional data on intraoral objects.

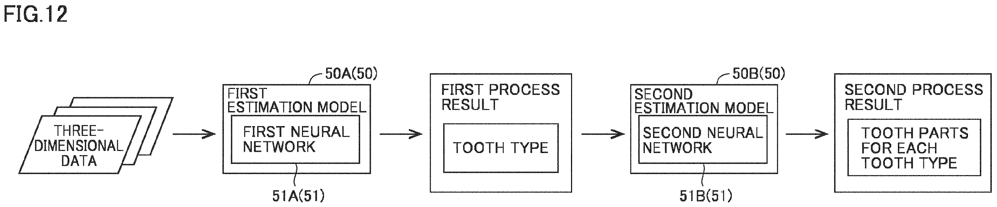

As shown in Fig. 12, data processing apparatus 10 may include, as estimation model 50, a first estimation model 50A including a first neural network 51A for identifying the type of a tooth, and a second estimation model 50B including a second neural network 51B for identifying a part of the tooth.

Specifically, data processing apparatus 10 may use first estimation model 50A to identify the type of a tooth based on the three-dimensional data indicating a surface shape of intraoral objects. In this case, first neural network 51A of first estimation model 50A has been trained by machine learning (for example, supervised learning) to identify the type of a tooth based on the directly input three-dimensional data on intraoral objects.

For example, in the training phase, first estimation model 50A finds features of the surface shape of an object indicated by three-dimensional data input to first neural network 51A, estimates the type of the tooth to which the three-dimensional data corresponds, and outputs the result of estimation. First estimation model 50A determines whether or not the output result of estimation matches the type of the tooth of correct answer data and, if they do not match, optimizes parameters 52 in such a manner that makes them match each other. Accordingly, in an operation phase, first estimation model 50A can estimate, with high precision, based on the three-dimensional data input to first neural network 51A, the type of the tooth corresponding to the three-dimensional data.

As a method of identifying the type of a tooth using first estimation model 50A based on the three-dimensional data indicating a surface shape of intraoral objects, data processing apparatus 10 adopts a method disclosed in Japanese Patent Laying-Open No. 2020-096691 (Japanese Patent No. 6650996). Therefore, regarding the identification of the type of tooth using an estimation model based on three-dimensional data indicating a surface shape of intraoral objects the disclosures of Japanese Patent Laying-Open No. 2020-096691 (Japanese Patent No. 6650996) are incorporated herein by reference.

Data processing apparatus 10 may use second estimation model 50B to identify a part of the tooth based on the three-dimensional data indicating a surface shape of intraoral objects associated with the type of the tooth estimated by first estimation model 50A. In this case, second neural network 51B of second estimation model 50B has been trained by machine learning (for example, supervised learning) to identify a part of the tooth based on the directly input three-dimensional data on intraoral objects associated with the type of the tooth.

For example, in the training phase, second estimation model 50B finds features of the surface shape of the object indicated by the three-dimensional data associated in advance with the type of the tooth input to second neural network 51B, estimates the part of the tooth to which the three-dimensional data corresponds, and outputs the result of estimation. Second estimation model 50B determines whether or not the output result of estimation matches the part of the tooth of correct answer data and, if they do not match, optimizes parameters 52 in such a manner that makes them match each other. Accordingly, in an operation phase, second estimation model 50B can estimate, with high precision, based on the three-dimensional data input to second neural network 51B, the part of the tooth corresponding to the three-dimensional data associated with the type of the tooth. Unlike estimation model 50 shown in Fig. 11, second estimation model 50B does not need to identify the type of the tooth, because the type of the tooth is associated in advance with the input three-dimensional data. Therefore, second estimation model 50B has an improved efficiency of the machine learning as compared with estimation model 50 shown in Fig. 11, and can estimate the part of the tooth with higher precision.

First estimation model 50A and second estimation model 50B shown in Fig. 12 may be different estimation models, or one estimation model 50 may have the functions of first estimation model 50A and second estimation model 50B as shown in Fig. 11.

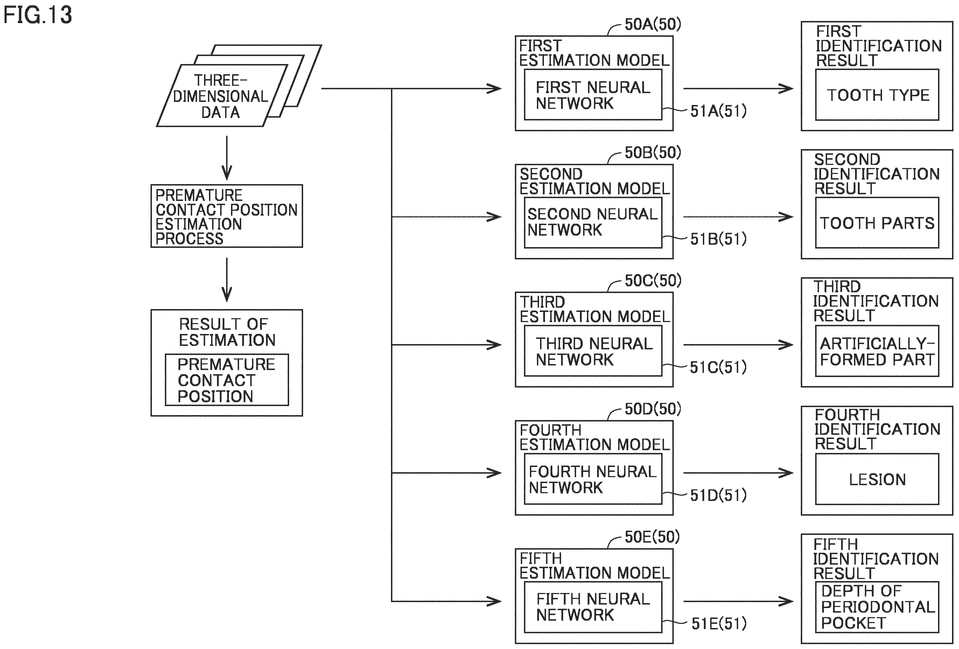

As shown in Fig. 13, data processing apparatus 10 may include, as estimation model 50, first estimation model 50A including first neural network 51A for identifying the type of a tooth, second estimation model 50B including second neural network 51B for identifying a part of the tooth, a third estimation model 50C including a third neural network 51C for identifying an artificially-formed part of the tooth, a fourth estimation model 50D including a fourth neural network 51D for identifying a lesion of the tooth, and a fifth estimation model 50E including a fifth neural network 51E for estimating the depth of a periodontal pocket in the tooth.

Specifically, data processing apparatus 10 may use first estimation model 50A to identify the type of a tooth based on the three-dimensional data indicating a surface shape of intraoral objects. In this case, as described with reference to Fig. 12, first neural network 51A of first estimation model 50A has been trained by machine learning (for example, supervised learning) to identify the type of a tooth based on the directly input three-dimensional data on intraoral objects.

Data processing apparatus 10 may use second estimation model 50B to identify a part of the tooth based on the three-dimensional data indicating a surface shape of intraoral objects. In this case, as described with reference to Figs. 7 to 10, second neural network 51B of second estimation model 50B has been trained by machine learning (for example, supervised learning) to identify a part of the tooth based on the directly input three-dimensional data on intraoral objects.

Data processing apparatus 10 may use third estimation model 50C to identify an artificially-formed part of the tooth based on the three-dimensional data indicating a surface shape of intraoral objects. In this case, third neural network 51C of third estimation model 50C has been trained by machine learning (for example, supervised learning) to identify an artificially-formed part of the tooth based on the directly input three-dimensional data on intraoral objects.

For example, in the training phase, third estimation model 50C finds features of the surface shape of the object indicated by the three-dimensional data input to third neural network 51C, estimates an artificially-formed part to which the three-dimensional data corresponds, and outputs the result of estimation. Third estimation model 50C determines whether or not the output result of estimation matches the artificially-formed part of the tooth of correct answer data and, if they do not match, optimizes parameters 52 in such a manner that make them match each other. Accordingly, in an operation phase, third estimation model 50C can estimate with high precision, based on the three-dimensional data input to third neural network 51C, an artificially-formed part of the tooth corresponding to the three-dimensional data.

As a method of identifying an artificially-formed part of a tooth using third estimation model 50C based on the three-dimensional data indicating a surface shape of intraoral objects, data processing apparatus 10 adopts a method disclosed in Japanese Patent Laying-Open No. 2022-012198 (Japanese Patent No. 7267974). Therefore, regarding the identification of artificially-formed parts of teeth using an estimation model based on three-dimensional data indicating a surface shape of intraoral objects the disclosures of Japanese Patent Laying-Open No. 2022-012198 (Japanese Patent No. 7267974) are incorporated herein by reference.

Data processing apparatus 10 may use fourth estimation model 50D to identify a lesion of the tooth based on the three-dimensional data indicating a surface shape of intraoral objects. In this case, fourth neural network 51D of fourth estimation model 50D has been trained by machine learning (for example, supervised learning) to identify a lesion of a tooth based on the directly input three-dimensional data on intraoral objects.

For example, in the training phase, fourth estimation model 50D finds features of the surface shape of the object indicated by the three-dimensional data input to fourth neural network 51D, estimates a lesion to which the three-dimensional data corresponds, and outputs the result of estimation. Fourth estimation model 50D determines whether or not the output result of estimation matches the lesion of the tooth of correct answer data and, if they do not match, optimizes parameters 52 in such a manner that makes them match each other. Accordingly, in an operation phase, fourth estimation model 50D can estimate, with high precision, based on the three-dimensional data input to fourth neural network 51D, the lesion of the tooth corresponding to the three-dimensional data.

As a method of identifying a lesion of a tooth using fourth estimation model 50D based on the three-dimensional data indicating a surface shape of intraoral objects, data processing apparatus 10 adopts a method disclosed in Japanese Patent Laying-Open No. 2022-012199 (Japanese Patent No. 7324735). Therefore, regarding the identification of a lesion of a tooth using an estimation model based on three-dimensional data indicating a surface shape of intraoral objects the disclosures of Japanese Patent Laying-Open No. 2022-012199 (Japanese Patent No. 7324735) are incorporated herein by reference.

Data processing apparatus 10 may use fifth estimation model 50E to estimate the depth of a periodontal pocket in the tooth, based on the three-dimensional data indicating a surface shape of intraoral objects. In this case, fifth neural network 51E of fifth estimation model 50E has been trained by machine learning (for example, supervised learning) to estimate the depth of a periodontal pocket in a tooth based on the directly input three-dimensional data on intraoral objects.

For example, in the training phase, fifth estimation model 50E finds features of the surface shape of an object indicated by three-dimensional data input to fifth neural network 51E, estimates the depth of a periodontal pocket to which the three-dimensional data corresponds, and outputs the result of estimation. Specifically, to fifth neural network 51E of fifth estimation model 50E, combined image data that is a combination of IOS data and CT data acquired for the same subject, and a predetermined measurement direction and a predetermined measurement point for measuring the depth of a periodontal pocket are input. Fifth estimation model 50E estimates the depth of the periodontal pocket based on these input data. Fifth estimation model 50E determines whether or not the output result of estimation matches the depth of the periodontal pocket of correct answer data and, if they do not match, optimizes parameters 52 in such a manner that makes them match each other. Accordingly, in an operation phase, fifth estimation model 50E can estimate, with high precision, based on the three-dimensional data input to fifth neural network 51E, the depth of the periodontal pocket in the tooth corresponding to the three-dimensional data.

As a method of estimating the depth of a periodontal pocket in a tooth using fifth estimation model 50E based on the three-dimensional data indicating a surface shape of intraoral objects, data processing apparatus 10 adopts a method disclosed in Japanese Patent Application No. 2023-013834. Therefore, regarding the estimation of a depth of a periodontal pocket using an estimation model based on three-dimensional data indicating a surface shape of intraoral objects the disclosures of Japanese Patent Application No. 2023-013834 are incorporated herein by reference.

Data processing apparatus 10 may estimate a premature contact position where the upper and lower dental arches make premature contact when they are occluded, by performing a process of estimating a premature contact position based on the three-dimensional data indicating a surface shape of intraoral objects.

For example, upper-jaw dental arch data indicating the surface shape of the dental arch of the upper jaw of the subject acquired by three-dimensional scanner 2 and lower-jaw dental arch data indicating the surface shape of the dental arch of the lower jaw of the subject acquired by three-dimensional scanner 2 are input to data processing apparatus 10. Data processing apparatus 10 uses a rendered image indicating the dental arch of the upper jaw generated based on the upper-jaw dental arch data and a rendered image indicating the dental arch of the lower jaw generated based on the lower-jaw dental arch data, to perform image processing so that the upper and lower dental arches change from the occluded state to the open state, and thereby calculate a premature contact position.

As a method of estimating a premature contact position based on the three-dimensional data indicating a surface shape of intraoral objects, data processing apparatus 10 adopts a method disclosed in Japanese Patent Application No. 2022-182611. Therefore, regarding the estimation of a premature contact position based on three-dimensional data indicating a surface shape of intraoral objects the disclosures of Japanese Patent Application No. 2022-182611 are incorporated herein by reference.

Thus, based on the three-dimensional data indicating a surface shape of intraoral objects, data processing apparatus 10 can estimate, besides the type of the tooth and the part of the tooth, at least one of an artificially-formed part of the tooth, a lesion of the tooth, the depth of a periodontal pocket in the tooth, and a premature contact position of teeth. First estimation model 50A for estimating the type of the tooth, second estimation model 50B for estimating the part of the tooth, third estimation model 50C for estimating an artificially-formed part of the tooth, fourth estimation model 50D for estimating a lesion of the tooth, and fifth estimation model 50E for estimating the depth of a periodontal pocket are identical to each other in that they use the three-dimensional data to calculate the result of estimation, and therefore, it is only necessary for data processing apparatus 10 to use the three-dimensional data common to these estimation models 50, to enable estimation of the type of the tooth, the part of the tooth, an artificially-formed part of the tooth, a lesion of the tooth, the depth of a periodontal pocket in the tooth, and a premature contact position of teeth all together. Then, data processing apparatus 10 can effectively utilize the results of estimation acquired by respective estimation models 50, to add, to a diagram or a table indicating the results of identification of the type of the tooth and the part of the tooth, information on at least one of the artificially-formed part, the lesion, the depth of the periodontal pocket, and the premature contact position. That is, the system used for each identification described above adopts the same coordinate system for the three-dimensional data, and the position coordinates included in respective results of identification are correct relative to each other. Therefore, data processing apparatus 10 can easily recognize the part and the type of the tooth having an artificially-formed part or a lesion, for example. In the case where respective results of identification acquired by systems adopting respective coordinate systems different from each other are used mutually, data processing apparatus 10 may perform a conversion process to make the position coordinates included in the results of identification correct relative to each other. As such a conversion process, alignment or registration of general three-dimensional data is used and, for example, a method such as ICP (Iterative Closest Point) may be adopted.

For example, data processing apparatus 10 may identify the type and the part of a tooth where an artificially-formed part is located, display the result in a diagram or a table, or output the result in the form of an electronic medical chart. Data processing apparatus 10 may identify the type and the part of a tooth where a lesion is located, display the result in a diagram or a table, or output the result in the form of an electronic medical chart. Data processing apparatus 10 may estimate the depth of a periodontal pocket for each type and each part of the tooth, display the result in a diagram or a table, or output the result in the form of an electronic medical chart. Data processing apparatus 10 may also estimate a premature contact position for each type and each part of the tooth, display the result in a diagram or a table, or output the result in the form of an electronic medical chart. Further, data processing apparatus 10 may combine a plurality of results selected from the artificially-formed part, the lesion, the depth of the periodontal pocket, and the premature contact position, associate the selected results with the type and the part of the tooth, display the results in a diagram or a table, or output the results in the form of an electronic medical chart.

Accordingly, it is unnecessary for the user to conduct, by him or herself, various examinations such as examinations for an artificially-formed part, a lesion, the depth of a periodontal pocket, or a premature contact position, and it is also unnecessary for the user to identify the type and the part of the examined tooth by him or himself. Therefore, the user can avoid taking time for a dental checkup or recording wrong information in a medical chart.

First estimation model 50A, second estimation model 50B, third estimation model 50C, fourth estimation model 50D, and fifth estimation model 50E shown in Fig. 13 may be estimation models different from each other, or one estimation model 50 may have respective functions of first estimation model 50A, second estimation model 50B, third estimation model 50C, fourth estimation model 50D, and fifth estimation model 50E. Moreover, data processing apparatus 10 may prepare a common estimation model from a plurality of estimation models included in first estimation model 50A, second estimation model 50B, third estimation model 50C, fourth estimation model 50D, and fifth estimation model 50E, so as to acquire different results of identification by one estimation model. For example, one common estimation model prepared from first estimation model 50A and second estimation model 50B may be used to estimate the type of a tooth and estimate a part of the tooth.

### [Modification of Process of Identification of Tooth Part]

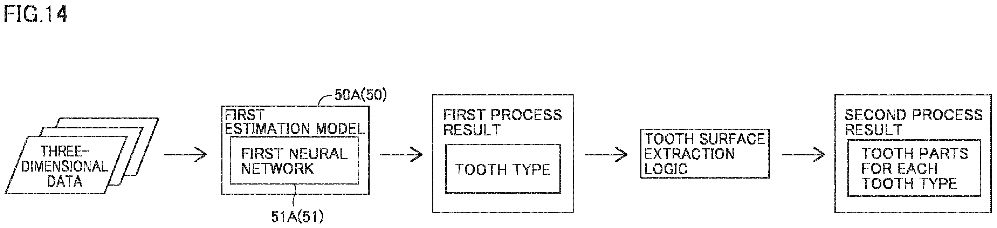

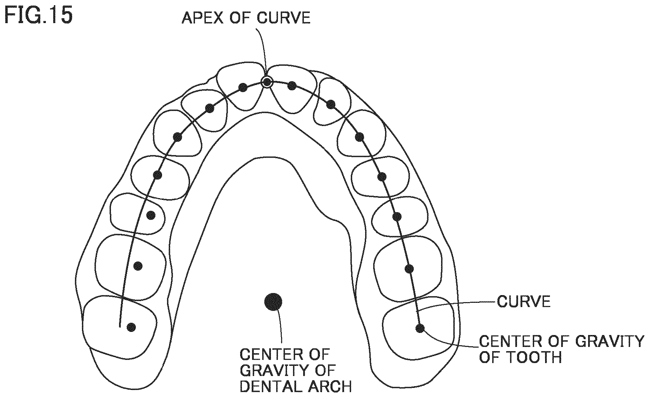

Fig. 14 is a diagram showing a modification of the process of identifying parts of teeth by data processing apparatus 10. As shown in Fig. 14, data processing apparatus 10 may estimate the type of a tooth using first estimation model 50A, and then estimate a part of the tooth, following a tooth surface extraction logic, without using second estimation model 50B illustrated in Fig. 12. In the following, an example of the identification process using the tooth surface extraction logic is described with reference to Figs. 15 to 28.

Figs. 15 to 28 are diagrams showing an example of the identification process by data processing apparatus 10 using the tooth surface extraction logic. Initially, with reference to Figs. 15 to 21, an example is described in which data processing apparatus 10 identifies a part of a tooth, for a canine, a lateral incisor, and a central incisor of the upper jaw or the lower jaw.

As shown in Fig. 15, based on three-dimensional data indicating the surface shape of the dental arch of the upper jaw or the lower jaw, data processing apparatus 10 generates a rendered image indicating the dental arch. In Fig. 15, the rendered image indicating the dental arch of the lower jaw is shown. Data processing apparatus 10 calculates the center of gravity of each of teeth constituting the dental arch. Data processing apparatus 10 draws a curve (or a polygonal line) passing through the center of gravity of each of the teeth in the rendered image. Data processing apparatus 10 calculates the center of gravity of the curve (i.e., the center of gravity of the dental arch). Data processing apparatus 10 may calculate the center of gravity of the tooth and the center of gravity of the dental arch, based on the coordinates of the three-dimensional data. Moreover, data processing apparatus 10 specifies the tip of the dental arch (the tip between the left central incisor and the right central incisor) in which the curve extends, as the apex of the curve.

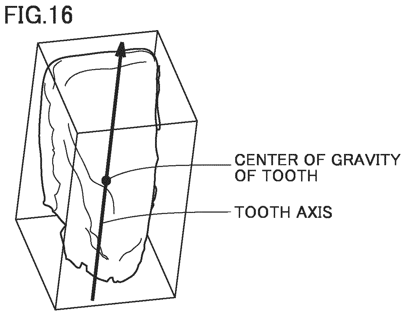

As shown in Fig. 16, data processing apparatus 10 extracts an image of one tooth from the teeth included in the dental arch shown in Fig. 15. In Fig. 16, an image of a lateral incisor included in the dental arch of the lower jaw is extracted. Data processing apparatus 10 calculates coordinates of a bounding box surrounding the tooth, generates the bounding box based on the calculated coordinates to surround the tooth. Data processing apparatus 10 calculates the center of gravity of the tooth surrounded by the bounding box. Data processing apparatus 10 generates an axis of the tooth that passes through the center of gravity of the tooth and extends in the direction of sides of the bounding box. For example, data processing apparatus 10 generates a first axis that passes through the center of gravity of the tooth and extends in the direction of the longer sides of the bounding box, and a second axis that passes through the center of gravity of the tooth and extends in the direction of the shorter sides of the bounding box. Data processing apparatus 10 specifies the longest axis (for example, the first axis) among the plurality of generated axes, as the tooth axis.

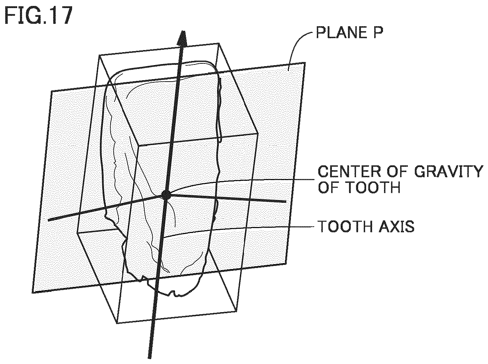

As shown in Fig. 17, data processing apparatus 10 specifies, as plane P, a plane extending in the direction of the curve of the dental arch (the curve shown in Fig. 15) among a plurality of planes passing through the center of gravity of the tooth.

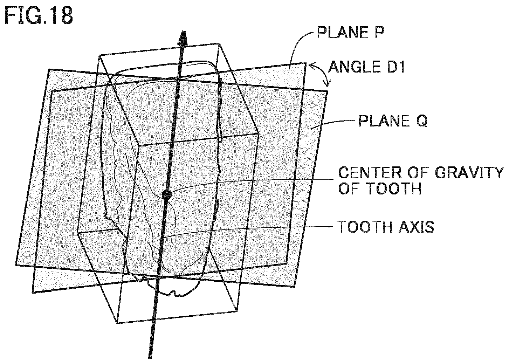

As shown in Fig. 18, data processing apparatus 10 generates a plane Q by rotating plane P by a predetermined angle D1 in a first direction of rotation about the tooth axis serving as the central axis. Angle D1 can be set by the user.

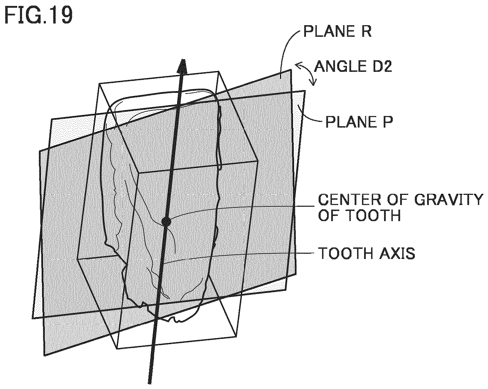

As shown in Fig. 19, data processing apparatus 10 generates a plane R by rotating plane P by a predetermined angle D2 in a second direction of rotation about the tooth axis as the central axis, opposite to the first direction of rotation. Angle D2 can be set by the user, and may be the same as or different from angle D1.

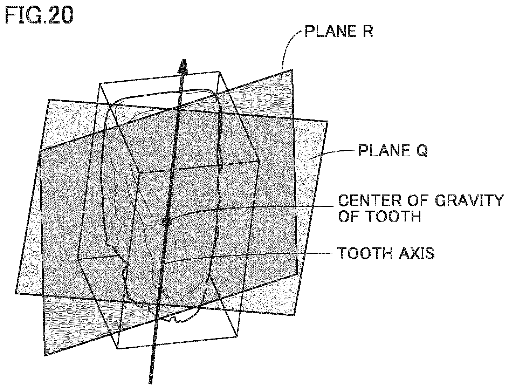

As shown in Fig. 20, data processing apparatus 10 can divide the surface of the tooth into four parts by dividing the tooth along each of the directions of plane R and plane Q.

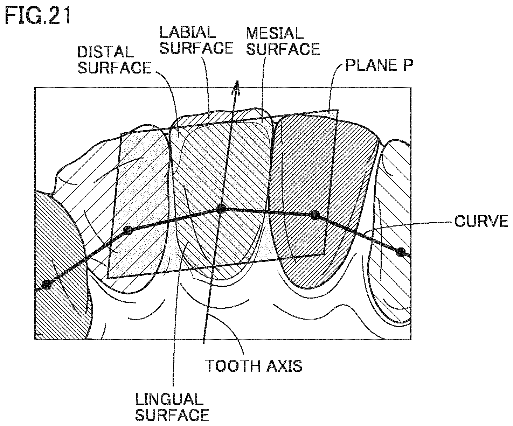

As shown in Fig. 21, data processing apparatus 10 allocates any of distal surface, mesial surface, labial surface, and lingual surface to each of the four surface parts into which the tooth (the lateral incisor in this example) is divided. For example, data processing apparatus 10 specifies, as the mesial surface, a surface that is closer to the apex of the curve of the dental arch and has many portions in contact with the adjacent tooth. Data processing apparatus 10 specifies, as the distal surface, a surface further from the apex of the curve of the dental arch and has many portions in contact with the adjacent tooth. Data processing apparatus 10 specifies, as the labial surface, a surface further from the center of gravity of the dental arch. Data processing apparatus 10 specifies, as the lingual surface, a surface closer to the center of gravity of the dental arch.

In Figs. 15 to 21 described above, an example of identifying parts of the surface of the lateral incisor of the lower jaw is illustrated, however, data processing apparatus 10 can also identify parts of each tooth by the method shown in Figs. 15 to 21, for any of canines and central incisors of the lower jaw, and canines, lateral incisors, and central incisors of the upper jaw as well.

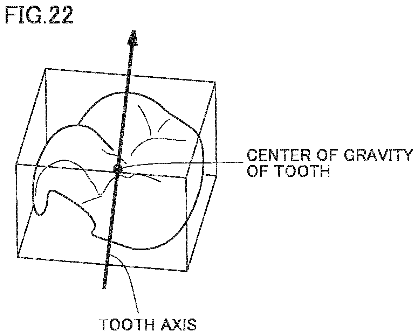

Next, with reference to Figs. 22 to 28, an example is described in which data processing apparatus 10 identifies a part of a tooth for a first premolar, a second premolar, a first molar, a second molar, and a third molar of the upper jaw or the lower jaw.

Initially, using the method shown in Fig. 15, data processing apparatus 10 draws a curve passing through the center of gravity of each of the teeth and calculates the center of gravity of the curve, in the rendered image indicating the dental arch generated based on the three-dimensional data indicating the surface shape of the dental arch of the upper jaw or the lower jaw. Moreover, data processing apparatus 10 specifies the tip of the dental arch (the tip between the left central incisor and the right central incisor) in which the curve extends, as the apex of the curve.

As shown in Fig. 22, data processing apparatus 10 extracts an image of one tooth from the teeth included in the dental arch shown in Fig. 15. In Fig. 22, an image of a first molar included in the dental arch of the lower jaw is extracted. Data processing apparatus 10 calculates coordinates of a bounding box surrounding the tooth, generates the bounding box based on the calculated coordinates to surround the tooth. Data processing apparatus 10 calculates the center of gravity of the tooth surrounded by the bounding box. Data processing apparatus 10 generates an axis of the tooth that passes through the center of gravity of the tooth and extends in the direction of sides of the bounding box. For example, data processing apparatus 10 generates a first axis that passes through the center of gravity of the tooth and extends in the direction of the longer sides of the bounding box, and a second axis that passes through the center of gravity of the tooth and extends in the direction of the shorter sides of the bounding box. Data processing apparatus 10 specifies the shortest axis (for example, the second axis) among the plurality of generated axes, as the tooth axis.

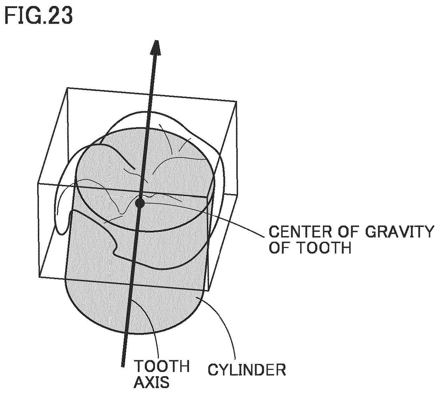

As shown in Fig. 23, data processing apparatus 10 applies a cylinder having a predetermined radius r so that its central axis corresponds to the tooth axis. Data processing apparatus 10 specifies a surface of the tooth included inside the cylinder as an occlusal surface. Radius r can be set by the user. Moreover, data processing apparatus 10 may apply an elliptical column or a prism to the tooth, instead of the cylinder.

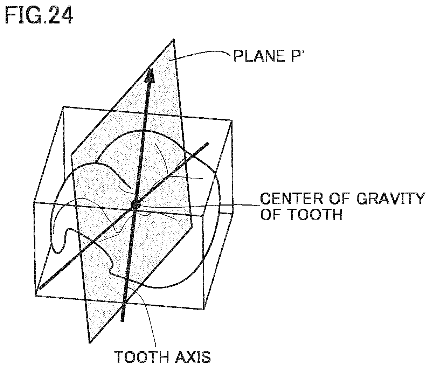

As shown in Fig. 24, data processing apparatus 10 specifies, as a plane P', a plane extending in the direction of the curve of the dental arch (the curve shown in Fig. 15) among a plurality of planes passing through the center of gravity of the tooth.

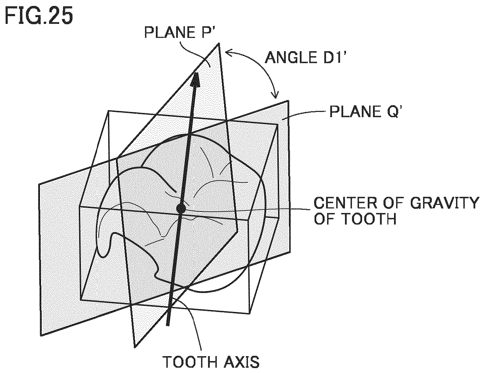

As shown in Fig. 25, data processing apparatus 10 generates a plane Q' by rotating plane P' by a predetermined angle D1' in the first direction of rotation about the tooth axis serving as the central axis. Angle D1' can be set by the user.

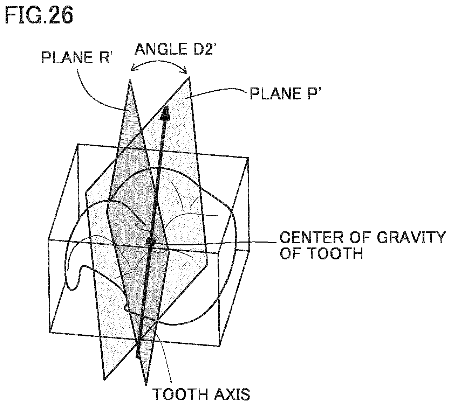

As shown in Fig. 26, data processing apparatus 10 generates a plane R' by rotating plane P' by a predetermined angle D2' in the second direction of rotation about the tooth axis serving as the central axis, opposite to the first direction of rotation. Angle D2' can be set by the user, and may be the same as or different from angle D1'.

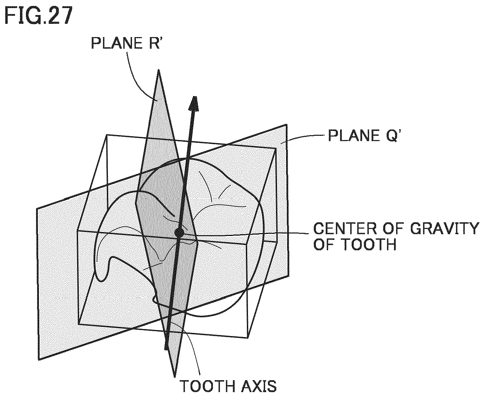

As shown in Fig. 27, data processing apparatus 10 can divide the surface of the tooth into four parts by dividing the tooth along each of the directions of plane R' and plane Q'.

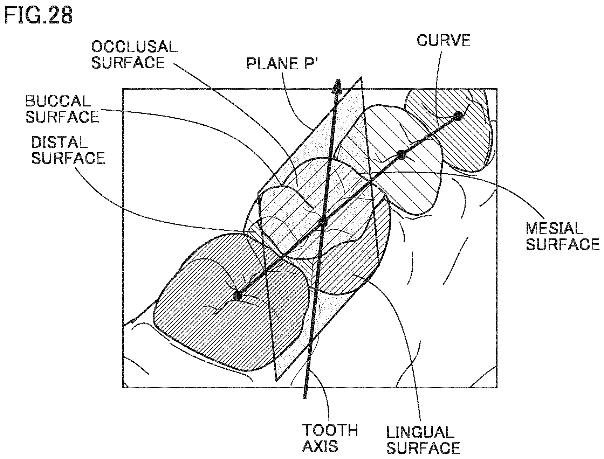

As shown in Fig. 28, data processing apparatus 10 allocates any of occlusal surface, distal surface, mesial surface, lingual surface, and buccal surface to each of the five surface parts into which the tooth (the first molar in this example) is divided. For example, data processing apparatus 10 specifies the surface determined by the cylinder shown in Fig. 23 as the occlusal surface. Data processing apparatus 10 specifies, as the mesial surface, a surface that is closer to the apex of the curve of the dental arch and has many portions in contact with the adjacent tooth. Data processing apparatus 10 specifies, as the distal surface, a surface that is further from the apex of the curve of the dental arch and has many portions in contact with the adjacent tooth. Data processing apparatus 10 specifies, as the buccal surface, a surface further from the center of gravity of the dental arch. Data processing apparatus 10 specifies, as the lingual surface, a surface closer to the center of gravity of the dental arch.

In Figs. 22 to 28 described above, an example of identifying parts of the surface of the first molar of the lower jaw is illustrated, however, data processing apparatus 10 can identify parts of each tooth by the method shown in Figs. 22 to 28, for any of the first molars, the second molars, the first premolars, and the second premolars of the lower jaw, and the first molars, the second molars, the third molars, the first premolars, and the second premolars of the upper jaw as well.

Thus, based on a plurality of parts into which the tooth is divided along a plurality of planes along the tooth axis passing through the center of gravity of the tooth, as well as the center of gravity of the dental arch including the tooth, data processing apparatus 10 can identify each of these plurality of parts. Further, data processing apparatus 10 can specify the occlusal surface of the tooth, using at least one of a cylinder, an elliptical column, and a prism having its central axis corresponding to the tooth axis. Accordingly, data processing apparatus 10 can estimate the parts of the tooth, following the tooth surface extraction logic, without using estimation model 50, and therefore, it is not necessary to train estimation model 50 by machine learning.

### [Data Processing]

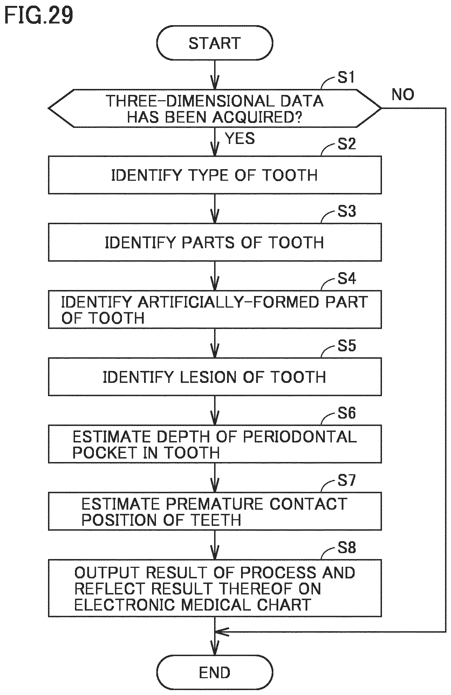

With reference to Fig. 29, data processing performed by data processing apparatus 10 is described. Fig. 29 is a flowchart defining the data processing performed by data processing apparatus 10. Each STEP (represented by "S" hereinafter) shown in Fig. 29 is implemented through execution of data processing program 100 by computing device 11 of data processing apparatus 10.

As shown in Fig. 29, data processing apparatus 10 determines whether or not three-dimensional data including three-dimensional position information corresponding to each of a plurality of points representing a surface shape of intraoral objects has been acquired (S1). When the three-dimensional data has not been acquired (NO in S1), data processing apparatus 10 ends this processing.

On the contrary, when the three-dimensional data has been acquired (YES in S1), data processing apparatus 10 identifies, based on the three-dimensional data, the type of the tooth on which the three-dimensional data has been acquired (S2). For example, data processing apparatus 10 directly inputs the three-dimensional data acquired in S1 to neural network 51 (or first neural network 51A) of estimation model 50 (or first estimation model 50A), to identify the type of the tooth on which the three-dimensional data has been acquired.

Data processing apparatus 10 identifies each of a plurality of parts forming the surface of the tooth, based on the three-dimensional data (S3). For example, data processing apparatus 10 directly inputs the three-dimensional data acquired in S1 to neural network 51 (or second neural network 51B) of estimation model 50 (or second estimation model 50B), to identify each of a plurality of parts forming the surface of the tooth on which the three-dimensional data has been acquired. Alternatively, data processing apparatus 10 directly inputs, to neural network 51 (or second neural network 51B) of estimation model 50 (or second estimation model 50B), the three-dimensional data to which the result of identification of the tooth type acquired in S2 is added, to thereby identify each of a plurality of parts forming the surface of the tooth on which the three-dimensional data has been acquired. Alternatively, following the tooth surface extraction logic described with reference to Figs. 15 to 28, data processing apparatus 10 identifies each of a plurality of parts forming the surface of the tooth on which the three-dimensional data has been acquired.

Data processing apparatus 10 identifies an artificially-formed part of the tooth, based on the three-dimensional data (S4). For example, data processing apparatus 10 directly inputs the three-dimensional data acquired in S1 to neural network 51 (or third neural network 51C) of estimation model 50 (or third estimation model 50C), to identify an artificially-formed part of the tooth on which the three-dimensional data has been acquired.

Data processing apparatus 10 identifies a lesion of the tooth based on the three-dimensional data (S5). For example, data processing apparatus 10 directly inputs the three-dimensional data acquired in S1 to neural network 51 (or fourth neural network 51D) of estimation model 50 (or fourth estimation model 50D), to identify a lesion of the tooth on which the three-dimensional data has been acquired.

Data processing apparatus 10 estimates the depth of a periodontal pocket in the tooth, based on the three-dimensional data (S6). For example, data processing apparatus 10 directly inputs the three-dimensional data acquired in S1 to neural network 51 (or fifth neural network 51E) of estimation model 50 (or fifth estimation model 50E), to estimate the depth of a periodontal pocket in the tooth on which the three-dimensional data has been acquired.

Data processing apparatus 10 estimates a premature contact position of teeth based on the three-dimensional data (S7). For example, data processing apparatus 10 divides the three-dimensional data acquired in S1 into upper-jaw dental arch data indicating the surface shape of the upper-jaw dental arch and lower-jaw dental arch data indicating the surface shape of the lower-jaw dental arch, and uses the upper-jaw dental arch data and the lower-jaw dental arch data to perform image processing so that the upper and lower dental arches change from the occluded state to the open state, and thereby estimate a premature contact position.

Data processing apparatus 10 may perform only the process in S3, or may perform at least one of the processes in S4 to S7, in addition to the process in S3. Moreover, data processing apparatus 10 may perform the processes in S2 and S3, or may perform at least one of the processes in S4 to S7 in addition to the processes in S2 and S3.

Data processing apparatus 10 outputs, to display 20, the result of the data processing obtained by the processes in S2 to S7, and reflects the result in an electronic medical chart (S8). Thereafter, data processing apparatus 10 ends the data processing. Data processing apparatus 10 may perform the processes in S2 to S8 in real time every time it acquires the three-dimensional data in S1 obtained during scanning with three-dimensional scanner 2, or may perform the processes in S2 to S8 on the condition that it has acquired a predetermined amount of three-dimensional data in S1.

### [Example of Electronic Medical Chart]

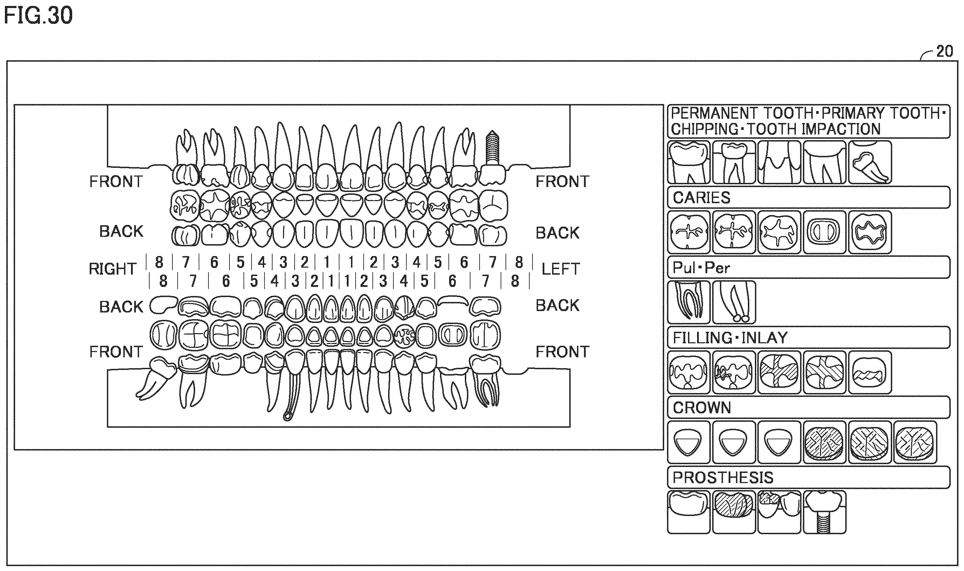

With reference to Figs. 30 to 35, an example of the electronic medical chart generated by data processing apparatus 10 is described. Figs. 30 to 35 are diagrams illustrating an example of the electronic medical chart generated by data processing apparatus 10. Data processing apparatus 10 reflects, on the electronic medical chart, the results of processing for the type of a tooth, a part of the tooth, an artificially-formed part, a lesion, the depth of a periodontal pocket, or a premature contact position obtained by the data processing, and presents, on display 20, a diagram or a table indicating details of the electronic medical chart. Here, reflecting each result of processing on the electronic medical chart means that data processing apparatus 10 recognizes and stores a correlation between an item corresponding to each part of each tooth type in a format of the electronic medical chart, and a part of each tooth type having been recognized, and further, based on the coordinate position of each part, recognizes and stores a correlation between the recognized part of the tooth type, and at least any one of the presence or absence or the type of an artificially-formed part, the presence or absence or the degree or the type of a lesion, the depth of a periodontal pocket, and the presence or absence or the degree of premature contact. Accordingly, based on the stored information, data processing apparatus 10 can present, on display 20, a diagram or a table indicating various states of each part of each tooth type.

For example, as shown in Fig. 30, data processing apparatus 10 aligns and displays teeth included in the dentition on display 20, and reflects the result of processing for an artificially-formed part, a lesion, the depth of a periodontal pocket, or a premature contact position for each tooth. In particular, data processing apparatus 10 reflects, on an electronic medical chart, a part having an artificially-formed part, a lesion, or the like, among a plurality of parts constituting the surface of a tooth, and presents, on display 20, an image indicating the result of reflection.

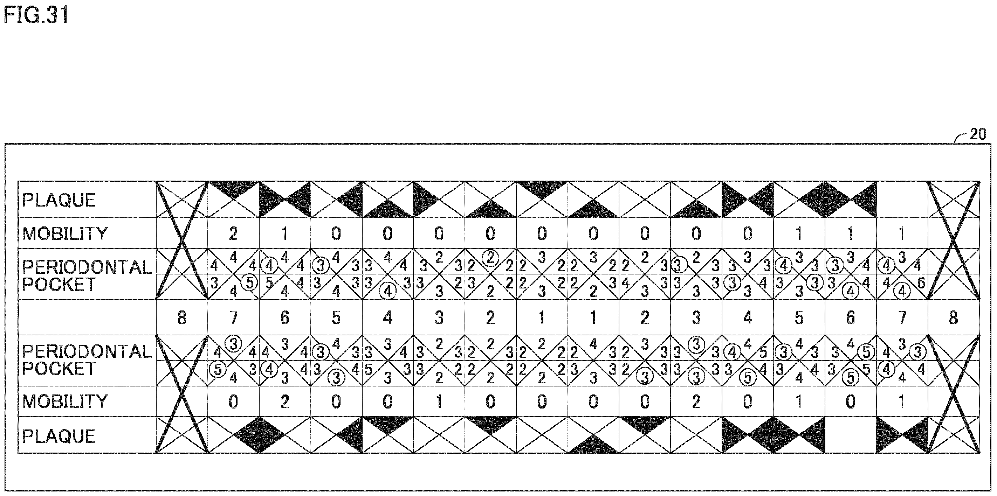

As shown in Fig. 31, data processing apparatus 10 may prepare a chart into which results of processing for the depth of a periodontal pocket in each tooth are complied, and present the prepared chart on display 20. In particular, data processing apparatus 10 compiles the results of processing for the depth of a periodontal pocket into the chart, for each of a plurality of parts forming the surface of a tooth, and presents the chart on display 20. Moreover, data processing apparatus 10 compiles, into a chart, information on the presence or absence of plaque (dental plaque) adhering to the surface of a tooth, for each of a plurality of parts forming the surface of the tooth, and presents the chart on display 20. Further, for each tooth, data processing apparatus 10 compiles, into a chart, the degree of mobility representing displacement of a tooth, and presents the chart on display 20. As for the degree of mobility, the result of measurement obtained by touching, by a dentist for example, of a tooth may be input to data processing apparatus 10.

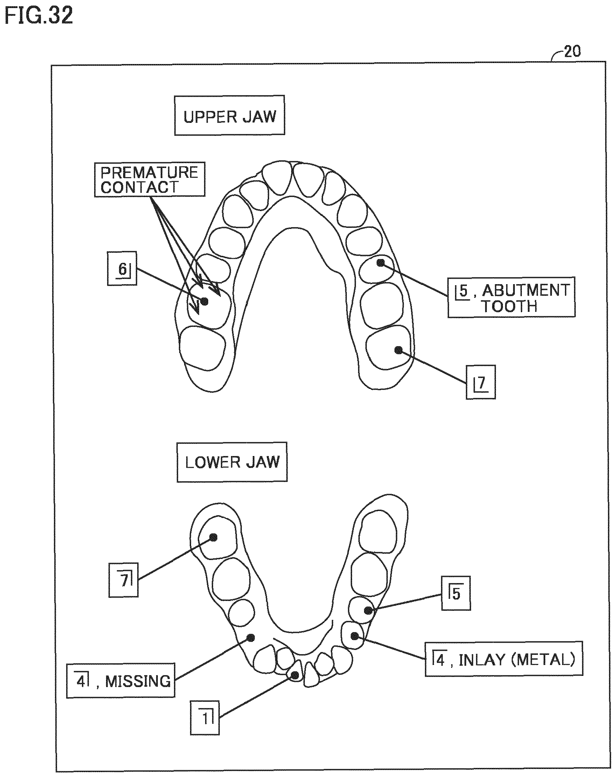

As shown in Fig. 32, data processing apparatus 10 may present, on display 20, at least one of the dental arch of the upper jaw and the dental arch of the lower jaw in three dimensions. Further, data processing apparatus 10 may add, to a tooth included in the dental arch presented in three dimensions, an annotation of the tooth number representing the type of the tooth, an annotation of an artificially-formed part such as inlay (metal) or abutment tooth, an annotation of a lesion such as chipping, or an annotation of a position of contact with the opposing tooth such as a part of premature contact position or a part of final contact position.

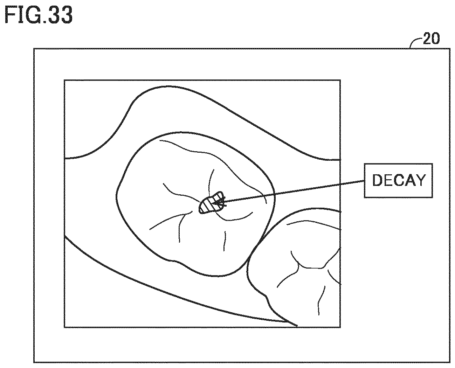

As shown in Fig. 33, data processing apparatus 10 may enlarge a portion of a dental arch and present a part of teeth in three dimensions on display 20. Further, data processing apparatus 10 may add an annotation of a lesion such as decay to a tooth presented in three dimensions.

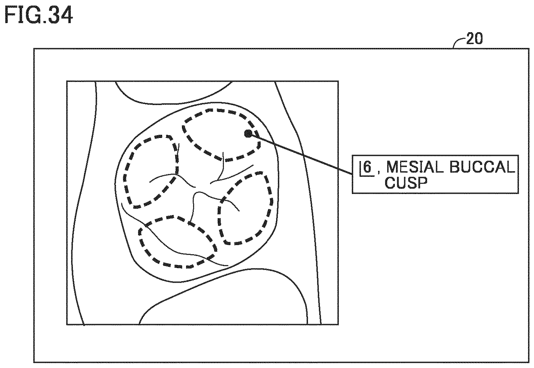

As shown in Fig. 34, data processing apparatus 10 may add, to a tooth presented in three dimensions, an annotation of identified positions of a plurality of cusps, for the first molar, the second molar, the third molar, the first premolar, or the second premolar.

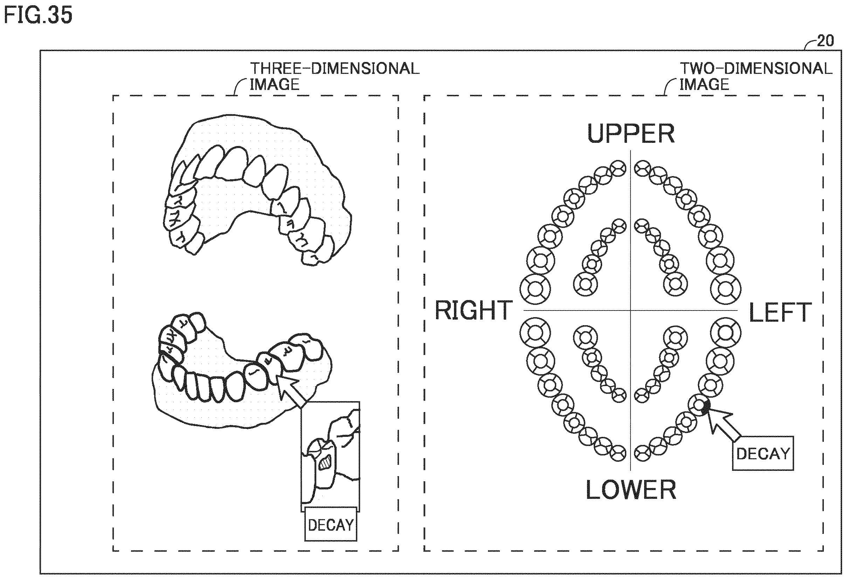

As shown in Fig. 35, data processing apparatus 10 may present, on display 20 side by side, a three-dimensional image indicating respective dental arches of the upper jaw and the lower jaw and a two-dimensional image indicating the dental formula. When an annotation is added by the user to a predetermined position of a tooth displayed in three dimensions, data processing apparatus 10 may add the annotation to the predetermined position of the tooth displayed in two dimensions. Further, when a cursor is placed by the user on the predetermined position of a tooth displayed in three dimensions, data processing apparatus 10 may highlight the predetermined position of the tooth displayed in two dimensions.

For example, in the case of the example of Fig. 35, when decay has occurred in the buccal surface of the second premolar of the lower jaw and data processing apparatus 10 receives from input device 30 an input to add an annotation of "decay" to the buccal surface of the second premolar shown in the three-dimensional image, it also adds the annotation of "decay" to the buccal surface of the second premolar shown in the two-dimensional image. Further, when data processing apparatus 10 receives from input device 30 an input to place a cursor on the buccal surface of the second premolar shown in the three-dimensional image, it also places the cursor on the buccal surface of the second premolar shown in the two-dimensional image to display an annotation of "decay" to thereby emphasize the buccal surface of the second premolar where the decay has occurred.

On the contrary, when an annotation is added by the user to a predetermined position of a tooth displayed in two dimensions, data processing apparatus 10 may add the annotation to the predetermined position of the tooth displayed in three dimensions. Further, data processing apparatus 10 may highlight a predetermined position of a tooth displayed in three dimensions, when a cursor is placed by the user on the predetermined position of the tooth displayed in two dimensions.

For example, receiving an input from input device 30 to add an annotation of "decay" to the buccal surface of the second premolar shown in the two-dimensional image, data processing apparatus 10 also adds the annotation of "decay" to the buccal surface of the second premolar shown in the three-dimensional image. Further, receiving from input device 30 an input to place a cursor on the buccal surface of the second premolar shown in the two-dimensional image, data processing apparatus 10 also places the cursor on the buccal surface of the second premolar shown in the three-dimensional image, and enlarges the buccal surface of the second premolar to display the annotation of "decay" to thereby emphasize the buccal surface of the second premolar where the decay has occurred. In the example of Fig. 35, data processing apparatus 10 presents, on display 20, an enlarged image of the buccal surface of the second premolar as seen in the direction of the buccal surface of the second premolar.

The highlighting on the two-dimensional image and the three-dimensional image shown in Fig. 35 is merely an example, and data processing apparatus 10 may highlight, by another method, the portion on which the cursor is placed. For example, receiving from input device 30 an input to place a cursor on the buccal surface of the second premolar shown in the two-dimensional image, data processing apparatus 10 may display, in a predetermined display region, an image obtained by cutting out the buccal surface of the second premolar shown in the three-dimensional image. When a cursor is placed on a specific tooth part in one of the two-dimensional image and the three-dimensional image, data processing apparatus 10 may highlight the specific tooth part in the other image by any of various methods such as changing the color of the specific tooth part, causing the specific tooth part to blink, enlarging the specific tooth part, or reducing the specific tooth part.

The user may use the QLF (Quantitative Light induced Fluorescence) method utilizing autofluorescence of teeth, to scan, with three-dimensional scanner 2, teeth irradiated with visible light. The user may scan, with three-dimensional scanner 2, a tooth discolored with a stain. Further, the user may scan, with three-dimensional scanner 2, a tooth discolored by sheet-like occlusal paper having a surface to which a paint in red or blue for example has been transferred.

As described above, data processing apparatus 10 may determine, based on the three-dimensional data on a tooth colored with visible light or a stain or the like in blue or the like, at least one of: whether or not a colored tooth part has a defect (for example, plaque), and the degree of defect. For example, estimation model 50 of data processing apparatus 10 has been trained by machine learning so as to be able to determine at least one of: whether or not a part of the tooth having been colored has a defect, and the degree of defect, based on the three-dimensional data on the colored tooth. Data processing apparatus 10 directly inputs the three-dimensional data on the colored tooth to neural network 51 of estimation model 50, to determine at least one of: whether the colored tooth part has a defect, and the degree of defect. Further, data processing apparatus 10 may associate an image (for example, the enlarged image shown in Fig. 35) indicating a colored tooth part, and an image (for example, an image of the "decay" shown in Fig. 35) indicating at least one of: the result of determination as to whether or not it has a defect, and the degree of defect, and present these images on display 20. When determining that a tooth part has a plaque as a defect of the tooth part, data processing apparatus 10 may present, on display 20, an image indicating whether each tooth has a plaque or not as shown in Fig. 31, for example. The determination as to whether a defect is present or not also includes any determination as to whether a defect is present or not that can be made based on the three-dimensional data on a tooth colored with visible light or stain or the like in blue or the like, such as determination as to whether a lesion such as decay has occurred, determination as to whether dental calculus adheres, and determination as to whether plaque adheres, for example. Moreover, the determination of the degree of defect also includes any determination of the degree of defect that can be made based on the three-dimensional data on a tooth colored with visible light or stain or the like in blue or the like, such as determination of the degree of lesion such as CO or C1 representing the degree of decay, and determination of the degree of adhesion of calculus. Determination as to whether or not a tooth has a defect or determination of the degree of defect can also be made by fourth estimation model 50D that identifies a lesion. For example, in the case where data processing apparatus 10 determines whether or not a plaque is present, fourth neural network 51D included in fourth estimation model 50D can determine whether or not a plaque is present, mainly based on features of the shape of the tooth part where a plaque has occurred, similarly to the determination for decay. At this time, fourth neural network 51D can determine, with higher precision, whether or not a plaque is present, by incorporating, in the input data, features such as the actual color of the tooth part or the degree of light. By using fourth estimation model 50D including fourth neural network 51D, data processing apparatus 10 can determine whether or not a plaque is present, without coloring the tooth with visible light or stain or the like in blue or the like, however, data processing apparatus 10 can also determine, with higher precision, whether or not a plaque is present, based on the three-dimensional data on the tooth colored with visible light or stain or the like in blue or the like. Thus, data processing apparatus 10 can use, as input data, the three-dimensional data on a tooth colored with visible light or stain or the like in blue or the like, to determine with higher precision whether or not a tooth part has a defect or the degree of defect. The estimation model 50 determines whether or not a defect is present or the degree of defect, in consideration of not only the color of the colored tooth but also the shape and the actual color of a defect, and the degree of light, or the like, and therefore, the precision of the determination is enhanced.

As described above, the three-dimensional data includes color information indicating the actual color of a portion (surface portion of an object) corresponding to each point of a point cloud representing a surface shape of intraoral objects. Then, data processing apparatus 10 may determine the actual color of each of a plurality of parts based on the three-dimensional data. For example, estimation model 50 of data processing apparatus 10 has been trained by machine learning so as to be able to determine the actual color of a tooth based on the three-dimensional data on the tooth including color information. Data processing apparatus 10 directly inputs the three-dimensional data on the tooth including the color information, to neural network 51 of estimation model 50, to determine the actual color of the tooth. Conventionally, in the case where a chipped tooth of a patient is to be treated with a ceramic prosthesis, practitioners such as dentists make visual check to determine the actual color of the tooth, using a predetermined color chart or the like. In contrast, data processing apparatus 10 can perform such determination using estimation model 50. Further, data processing apparatus 10 may associate an image indicating each tooth part with an image indicating the result of determination of the actual color of the tooth, to present these images on display 20. Moreover, data processing apparatus 10 may estimate, based on the three-dimensional data, which color in a color chart is the actual color of each of a plurality of parts. Accordingly, for example, for treating a patient's chipped tooth with a ceramic prosthesis, a dentist or the like can appropriately select a prosthesis of the same color as that of the patient's tooth.

As described above, data processing apparatus 10 can use estimation model 50 to identify the type of each tooth included in the dentition, and surface parts of each tooth. Then, data processing apparatus 10 may draw, on the dental arch of the lower jaw, a triangle by connecting the distal buccal cusp of the second molar on the left side, the distal buccal cusp of the second molar on the right side, and the midpoint (incisor point) of the mesial surfaces of the left and right central incisors, define this triangle as an occlusal plane, and output, to display 20, image data for presenting the occlusal plane. In this way, data processing apparatus 10 can present, on display 20, the imaginary occlusal plane defined by respective positions of the cusp of the left first molar, the cusp of the right first molar, and the central incisors identified by estimation model 50.

Thus, data processing apparatus 10 can automatically recognize the type of a tooth, parts of the tooth, an artificially-formed part, a lesion, the depth of a periodontal pocket, and a premature contact position, for example, and reflect them in an electronic medical chart, only by acquiring the three-dimensional data on teeth obtained by three-dimensional scanner 2, CT imaging device, or the like, so that the convenience for the user such as practitioner can be improved and the precision of a dental checkup can also be enhanced.

It should be construed that the embodiments disclosed herein are given by way of illustration in all respects, not by way of limitation. It is intended that the claimed subject-matter is defined by claims, not by the description above, and encompasses all modifications and variations of the claimed subject-matter. Features illustrated in connection with the embodiments and features illustrated in connection with the modifications may be combined as appropriate.

## Claims

1. A data processing apparatus configured to process three-dimensional data, the data processing apparatus comprising:
an input unit configured to acquire the three-dimensional data including three-dimensional position information corresponding to each of a plurality of points representing a surface shape of intraoral objects including a tooth;
a computing unit configured to perform a process for identifying each of a plurality of parts of the tooth, based on the three-dimensional data acquired by the input unit; and
an output unit configured to output a result of the process performed by the computing unit.

2. The data processing apparatus according to claim 1, wherein
in a case where the tooth is a first molar, a second molar, a third molar, a first premolar, or a second premolar of upper jaw, the plurality of parts include at least one of an occlusal surface, a distal surface, a mesial surface, a palatal surface, and a buccal surface of the tooth,
in a case where the tooth is a canine, a lateral incisor, or a central incisor of the upper jaw, the plurality of parts include at least one of a distal surface, a mesial surface, a palatal surface, and a labial surface of the tooth,
in a case where the tooth is a first molar, a second molar, a third molar, a first premolar, or a second premolar of lower jaw, the plurality of parts include at least one of an occlusal surface, a distal surface, a mesial surface, a lingual surface, and a buccal surface of the tooth, and
in a case where the tooth is a canine, a lateral incisor, or a central incisor of the lower jaw, the plurality of parts include at least one of a distal surface, a mesial surface, a lingual surface, and a labial surface of the tooth.

3. The data processing apparatus according to claim 1 or 2, wherein in a case where the tooth is a first molar, a second molar, a third molar, a first premolar, or a second premolar, the plurality of parts include respective cusps constituting the tooth.

4. The data processing apparatus according to anyone of the preceding claims, wherein in a case where the tooth is a canine, a lateral incisor, or a central incisor, the plurality of parts include a plurality of surfaces constituting a labial surface of the tooth.

5. The data processing apparatus according to anyone of the preceding claims, wherein the computing unit is configured to identify a tooth to which each of the plurality of parts belong, among teeth of upper jaw and lower jaw.

6. The data processing apparatus according to any one of the preceding claims, wherein the computing unit is configured to identify a type of the tooth, based on the three-dimensional data acquired by the input unit, and a first estimation model having undergone machine learning.

7. The data processing apparatus according to claim 6, wherein the first estimation model includes a first neural network having undergone machine learning to identify the type of the tooth based on the three-dimensional data input to the first neural network.

8. The data processing apparatus according to any one of the preceding claims, wherein the computing unit is configured to identify each of the plurality of parts, based on the three-dimensional data acquired by the input unit, and a second estimation model having undergone machine learning.

9. The data processing apparatus according to claim 8, wherein the second estimation model includes a second neural network having undergone machine learning to identify each of the plurality of parts, based on the three-dimensional data input to the second neural network.

10. The data processing apparatus according to any one of the preceding claims, wherein the computing unit is configured to identify an artificially-formed part of the tooth, based on the three-dimensional data acquired by the input unit, and a third estimation model having undergone machine learning.

11. The data processing apparatus according to claim 10, wherein the third estimation model includes a third neural network having undergone machine learning to identify the artificially-formed part, based on the three-dimensional data input to the third neural network.

12. The data processing apparatus according to any one of the preceding claims, wherein the computing unit is configured to identify a lesion of the tooth or gum, based on the three-dimensional data acquired by the input unit, and a fourth estimation model having undergone machine learning.

13. The data processing apparatus according to claim 12, wherein the fourth estimation model includes a fourth neural network having undergone machine learning to identify the lesion, based on the three-dimensional data input to the fourth neural network.

14. The data processing apparatus according to any one of the preceding claims, wherein the computing unit is configured to estimate a depth of a periodontal pocket in the tooth, based on the three-dimensional data acquired by the input unit, and a fifth estimation model having undergone machine learning.

15. The data processing apparatus according to claim 14, wherein the fifth estimation model includes a fifth neural network having undergone machine learning to estimate the depth of the periodontal pocket, based on the three-dimensional data input to the fifth neural network.

16. The data processing apparatus according to any one of the preceding claims, wherein the computing unit is configured to estimate a premature contact position where upper and lower dental arches make premature contact when the upper and lower dental arches are occluded, based on the three-dimensional data acquired by the input unit.

17. The data processing apparatus according to any one of the preceding claims, wherein the computing unit is configured to identify each of the plurality of parts, based on the plurality of parts into which the tooth is divided by a plurality of planes along a tooth axis passing through a center of gravity of the tooth, and based on a center of gravity of a dental arch including the tooth.

18. The data processing apparatus according to claim 17, wherein the computing unit is configured to identify an occlusal surface of the tooth, using at least one of a cylinder, an elliptical column, and a prism of which central axes are each the tooth axis.

19. The data processing apparatus according to any one of the preceding claims, wherein the output unit is configured to output image data for displaying a diagram or a table indicating the result of the process.

20. The data processing apparatus according to claim 19, wherein the result of the process is used for an electronic medical chart.

21. The data processing apparatus according to claim 19 or 20, wherein the image data includes data for displaying the tooth, together with the result of the process, in at least one of two dimensions and three dimensions.

22. The data processing apparatus according to claim 21, wherein
the image data includes data for displaying the tooth in two dimensions and three dimensions, and
in a case where an annotation is added by a user to a predetermined position of the tooth displayed in three dimensions, the annotation is added to the predetermined position of the tooth displayed in two dimensions.

23. The data processing apparatus according to claim 21 or 22, wherein
the image data includes data for displaying the tooth in two dimensions and three dimensions, and
in a case where an annotation is added by a user to a predetermined position of the tooth displayed in two dimensions, the annotation is added to the predetermined position of the tooth displayed in three dimensions.

24. The data processing apparatus according to any one of claims 21 to 23, wherein
the image data includes data for displaying the tooth in two dimensions and three dimensions, and
in a case where a cursor is placed by a user on a predetermined position of the tooth displayed in three dimensions, the predetermined position of the tooth displayed in two dimensions is highlighted.

25. The data processing apparatus according to any one of claims 21 to 24, wherein
the image data includes data for displaying the tooth in two dimensions and three dimensions, and
in a case where a cursor is placed by a user on a predetermined position of the tooth displayed in two dimensions, the predetermined position of the tooth displayed in three dimensions is highlighted.

26. The data processing apparatus according to any one of the preceding claims, wherein the result of the process includes information on a color associated with each of the plurality of parts.

27. The data processing apparatus according to any one of the preceding claims, wherein
the computing unit is configured to determine at least one of: whether a part of the tooth having been colored has a defect, and a degree of defect of the part, based on the three-dimensional data acquired by the input unit, and
the computing unit is configured to associate, based on the result of the process, the part of the colored tooth, with a result of determining at least one of: whether a part of the tooth having been colored has a defect, and a degree of defect of the part.

28. The data processing apparatus according to any one of the preceding claims, wherein
the computing unit is configured to determine an actual color of each of the plurality of parts, or a color, in a color chart, of each of the plurality of parts, based on the three-dimensional data acquired by the input unit, and
the computing unit is configured to associate, based on the result of the process, each of the plurality of parts, with a result of the determination of the actual color or the color, in the color chart, of each of the plurality of parts.

29. The data processing apparatus according to any one of the preceding claims, wherein
the result of the process includes respective positions of a distal buccal cusp of a second molar of a left side of lower jaw, a distal buccal cusp of a second molar of a right side of lower jaw, and mesial surfaces of a left central incisor and a right central incisor of lower jaw, and
the output unit is configured to output image data for displaying an occlusal plane generated based on the distal buccal cusp of the second molar of the left side of lower jaw, the distal buccal cusp of the second molar of the right side of lower jaw, and a midpoint of the mesial surfaces of the left central incisor and the right central incisor of lower jaw.

30. The data processing apparatus according to any one of the preceding claims, wherein
the three-dimensional data is three-dimensional scanner data acquired through scanning of the tooth using a three-dimensional scanner, and
the output unit is configured to output, in real time, the result of the process acquired during scanning with the three-dimensional scanner.

31. The data processing apparatus according to any one of the preceding claims, wherein the three-dimensional data includes at least one of three-dimensional scanner data acquired by scanning the tooth using a three-dimensional scanner, Cone Beam Coherence Tomography, CBCT, data acquired by imaging the tooth using cone beam Computed Tomography, CT, and Optical Coherence Tomography, OCT, data acquired by imaging the tooth using an optical coherence tomography device.

32. A data processing method of processing three-dimensional data by a computer, the data processing method comprising, as processing performed by the computer:
acquiring the three-dimensional data including three-dimensional position information corresponding to each of a plurality of points representing a surface shape of intraoral objects including a tooth;
performing a process for identifying each of a plurality of parts of the tooth, based on the three-dimensional data acquired in the acquiring; and
outputting a result of the process in the performing.

33. A data processing program for processing three-dimensional data by a computer, the data processing program causing the computer to perform:
acquiring the three-dimensional data including three-dimensional position information corresponding to each of a plurality of points representing a surface shape of intraoral objects including a tooth;
performing a process for identifying each of a plurality of parts of the tooth, based on the three-dimensional data acquired in the acquiring; and
outputting a result of the process in the performing.
